# FASCICULE DE BREVET EUROPEEN

(11) **EP 0 591 057 B1**
(45) Date de publication et mention de la délivrance du brevet: **10.07.1996**
(21) Numéro de dépôt: 93402394.6
(22) Date de dépôt: 01.10.1993
(51) Int. Cl.: C07C 233/18, A61K 31/16, C07C 233/60, C07C 233/20, C07D 319/18, A61K 31/335, C07D 307/79, A61K 31/34, C07D 209/08, A61K 31/40

(54) **Arylalkyl(thio)amides ayant une affinité sélective pour les récepteurs de la mélatonine et leur procédé de préparation**
Arylalkyl(thio)amide mit Melatonin-Rezeptor-Selektivität und Prozess zu ihrer Darstellung
Arylalkyl(thio)amides with melatonine receptor selectivity and process for their preparation

(30) Priorité: 02.10.1992 FR 9211671
(43) Date de publication de la demande: 06.04.1994
(73) Titulaire: ADIR ET COMPAGNIE, F-92415 Courbevoie Cédex (FR)
(72) Inventeur: Langlois, Michel, F-92330 Sceaux (FR); Renard, Pierre, F-78000 Versailles (FR); Adam, Gérard, F-78600 Le Mesnil le Roi (FR)

(56) Documents cités:
- EP-A- 0 447 285
- JOURNAL OF MEDICINAL CHEMISTRY vol. 22, no. 1 , 1979 , WASHINGTON US pages 63 - 69 M. E. FLAUGH ET AL. 'Synthesis and Evaluation of the Antiovulatory Activity of a Variety of Melatonin Analogues'
- JOURNAL OF HETEROCYCLIC CHEMISTRY vol. 15 , Décembre 1978 , PROVO US pages 1351 - 1359 E. CAMPAIGNE ET AL. 'Benzo[b]thiophene Derivatives. XXIII Derivatives of 5,6-Dihydroxy-3-benzo[b]thienylacetic Acid (1)'

## Description

La demande concerne de nouveaux arylalkyl(thio)amides, leur procédé de préparation, et les compositions pharmaceutiques qui les contiennent.

La demande EP 238 868 a déjà décrit des composés de structure phénylalkylamidique en les présentant comme inhibiteurs de tyrosine-kinase, antitumoraux et antibactériens.

De nombreuses études ont mis en évidence le rôle capital de la mélatonine (5-méthoxy N-acétyl tryptamine) dans le contrôle du rythme circadien et des fonctions endocrines, et les récepteurs de la mélatonine ont été caractérisés et localisés. L'obtention de composés actifs sur le système mélatoninergique constitue donc un réel besoin pour le praticien.

La demanderesse a découvert que de nouveaux composés de structure arylalkyl(thio)amides, substitués sur leur noyau benzène par un radical hydroxyle ou alcoxy spécifiquement en position ortho de la chaîne alkyl(thio)amidique, possèdaient une activité très importante sur le système mélatoninergique, alors qu'on ne retrouve pas ces propriétés avec les composés substitués en position méta ou para de la chaîne alkyl(thio)amidique.

Plus particulièrement, la présente invention concerne les composés de formule (I) : 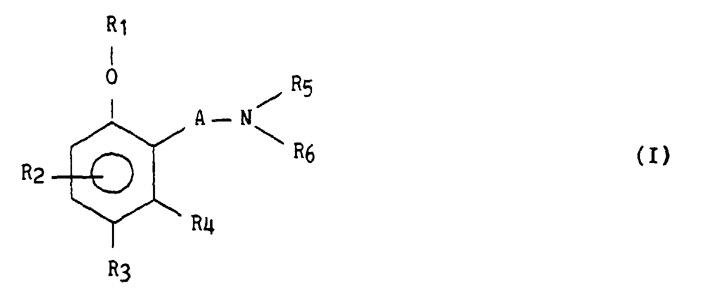 dans laquelle :
- A représente une chaîne -CH₂-CH₂- non substituée ou substituée par un alkyle inférieur,
- R₁ représente un atome d'hydrogène ou un alkyle inférieur,
- R₂ représente un atome d'hydrogène, un atome d'halogène, ou un alkyle inférieur,
- R₃ et R₄, identiques ou différents, représentent chacun indépendamment l'un de l'autre, un radical choisi parmi :
   . hydrogène,
   . halogène,
   . alkyle inférieur,
   . cyano,
   . carboxyle,
   . nitro,
   . amino,
   . alkylamino inférieur,
   . dialkylamino inférieur,
   . -(CH₂)ₙ-E₁ dans lequel n représente 0 ou un nombre entier de 1 à 4, et E₁ représente un cycloalkyle ou un cycloalcényle et contient de 3 à 8 atomes de carbone, E₁ étant non substitué ou substitué par un ou plusieurs radicaux choisis parmi halogène, oxo, et alkyle inférieur,
   . et -(CH₂)_{n'}-E₂ dans lequel n' représente 0 ou un nombre entier de 1 à 4 et E₂ représente un radical choisi parmi : phényle, naphtyle, pyrrolyle, thiényle, furyle, pyridyle, pyrazinyle, pyrimidinyle, pyridazinyle, quinolyle, et isoquinolyle, E₂ étant non substitué ou substitué par un ou plusieurs radicaux choisis parmi : halogène, hydroxyle, alkyle inférieur, alcoxy inférieur, et trifluorométhyle,
   ou R₃ et R₄ forment ensemble avec le noyau benzène qui les porte un système cyclique E₃ choisi parmi : indène, naphtalène, benzothiophène, benzofurane, indole, benzimidazole, benzopyrane, benzothiopyrane, quinoléine, isoquinoléine, indazole, quinoxaline, quinazoline, cinnoline, benzothiazole, benzisothiazole, benzoxazole, benzisoxazole, benzoxazine, benzothiazine, benzodioxole, et benzodioxane,
   étant entendu que la partie du système cyclique E₃ formée par R₃ et R₄ et les 2 atomes de carbone du noyau benzène qui les portent est :
- non hydrogénée ou partiellement hydrogénée,
- et non substituée ou substituée par un ou plusieurs radicaux choisis parmi : halogène, hydroxyle, alkyle inférieur, alcoxy inférieur, alcoxycarbonyle inférieur, et trifluorométhyle,
- R₅ représente un atome d'hydrogène ou un alkyle inférieur,
- R₆ représente un groupement 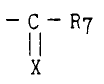 dans lequel X représente un atome de soufre ou d'oxygène et R₇ représente un radical choisi parmi :
   . alkyle inférieur non substitué ou substitué par un ou plusieurs radicaux choisis parmi halogène, hydroxy, et alcoxy inférieur,
   . alcènyle linéaire ou ramifié de 2 à 7 atomes de carbone non substitué ou substitué par un ou plusieurs radicaux choisis parmi halogène, hydroxy, et alcoxy inférieur,
   . et -(CH₂)ₘ-E₄ dans lequel m représente 0 ou un nombre entier de 1 à 4 et E₄ représente un cycloalkyle, mono ou bicyclique, de 3 à 12 atomes de carbone non substitué ou substitué par un ou plusieurs radicaux choisis parmi halogène, oxo, et alkyle inférieur,
   avec la réserve que R₇ peut représenter un alkyle inférieur non substitué ou substitué par un ou plusieurs radicaux choisis parmi halogène, hydroxy, et alcoxy inférieur, que si R₃ et R₄ forment avec le noyau benzène qui les porte un système cyclique E₃ tel que défini précédemment ou si un des substituants R₃ et R₄ représente un alkyle inférieur et que l'autre des substituants R₃ et R₄ représente un atome d'hydrogène,
   leurs isomères optiques,
   et leurs sels d'addition à un acide ou à une base pharmaceutiquement acceptable lorsque R₃ ou R₄ représente un groupement salifiable,
   étant entendu que les termes "alkyle inférieur" et "alcoxy inférieur" désignent des groupements linéaires ou ramifiés de 1 à 6 atomes de carbone et que les termes "cycloalcènyle" et "alcènyle" désignent des groupements hydrocarbonés contenant une ou plusieurs double liaisons.

L'invention s'étend également :
- aux composés selon la formule (I) dans lesquels R₃ et R₄ forment ensemble avec le noyau benzène qui les porte un système cyclique E₃ choisi parmi : indène, naphtalène, benzothiophène, benzofurane, indole, benzimidazole, benzopyrane, benzothiopyrane, quinoléine, isoquinoléine, indazole, quinoxaline, quinazoline, cinnoline, benzothiazole, benzisothiazole, benzoxazole, benzisoxazole, benzoxazine, benzothiazine, benzodioxole, et benzodioxane,
   étant entendu que la partie du système cyclique E₃ formée par R₃ et R₄ et les 2 atomes de carbone du noyau benzène qui les portent est :
   . non hydrogénée ou partiellement hydrogénée,
   . et non substituée ou substituée par un ou plusieurs radicaux choisis parmi : halogène, hydroxyle, alkyle inférieur, alcoxy inférieur, alcoxycarbonyle inférieur, et trifluorométhyle,
- aux composés selon la formule (I) dans lesquels R₃ et R₄ forment ensemble avec le noyau benzène qui les porte un naphtalène,
   étant entendu que le noyau benzène formé par R₃, R₄, et les 2 atomes de carbone qui les portent est non substitué ou substitué par un ou plusieurs radicaux choisis parmi halogène, hydroxyle, alkyle inférieur, alcoxy inférieur, alcoxycarbonyle inférieur et trifluorométhyle,
- et aux composés selon la formule (I) dans lesquels R₁ représente un radical méthyle, et R₃ et R₄ forment ensemble avec le noyau benzène qui les porte un naphtalène étant entendu que le noyau benzène formé par R₃ et R₄ et les 2 atomes de carbone qui les portent est non substitué ou substitué par un ou plusieurs radicaux choisis parmi : halogène, hydroxyle, alkyle inférieur, alcoxy inférieur, alcoxycarbonyle inférieur, et trifluorométhyle.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour salifier les composés utilisés selon l'invention, on peut citer, à titre d'exemples et de façon non limitative la soude, la potasse, la triéthylamine, la diéthylamine, l'éthanolamine, l'arginine, la lysine, et la diéthanolamine.

L'invention s'étend également au procédé de préparation des composés de formule (I),
caractérisé en ce que :
on fait réagir un composé de formule (II) : 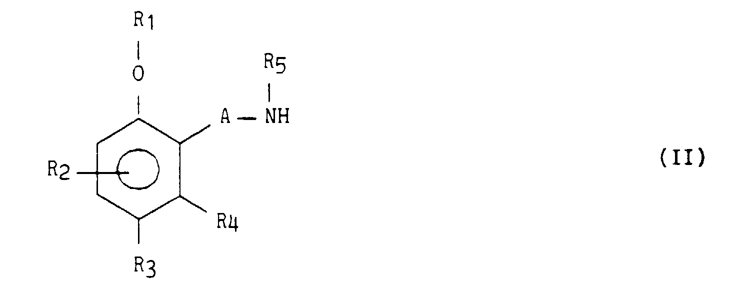 dans laquelle A, R₁, R₂, R₃, R₄ et R₅ sont tels que définis dans la formule (I),
avec un composé de formule (III) : 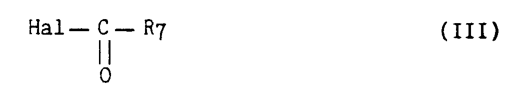 ou de formule (IV) : 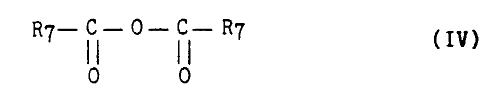 dans lesquelles R₇ est tel que défini dans la formule (I) et Hal représente un atome d'halogène, pour obtenir un composé de formule (I/a) : 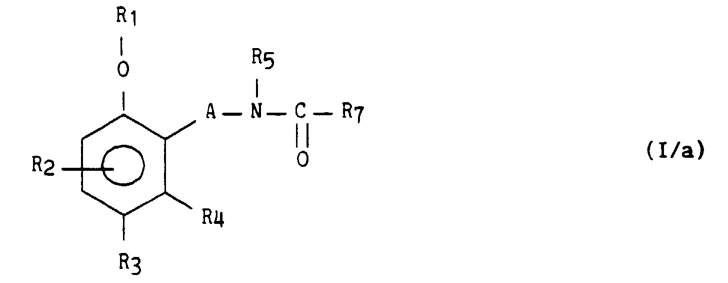 dans laquelle A, R₁, R₂, R₃, R₄, R₅, et R₇ sont tels que définis précédemment,
composé de formule (I/a) qui est soumis au réactif de Lawesson afin d'obtenir le composé de formule (I/b) : 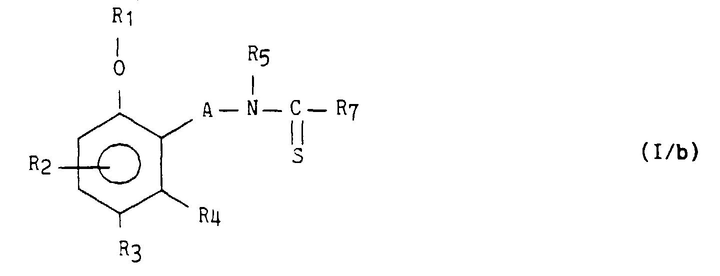 dans laquelle A, R₁, R₂, R₃, R₄, R₅, et R₇ sont tels que définis précédemment,
les composés de formule (I/a) et (I/b) formant l'ensemble des composés de formule (I),
les composés de formule (I) pouvant être :
- purifiés suivant une ou plusieurs méthodes de purification choisies parmi la cristallisation, la chromatographie sur colonne de silice, la chromatographie en phase gazeuse, l'extraction, la filtration, et le passage sur charbon ou résine,
- séparés, le cas échéant, sous forme pure ou sous forme de mélange, en leurs éventuels isomères optiques,
- ou salifiés par un acide ou une base pharmaceutiquement acceptable.

Les composés de départ utilisés dans le procédé de préparation des composés de formule (I) sont :
- soit commerciaux,
- soit aisément accessibles à l'homme de l'art par des procédés contenus dans la littérature.

Les composés de formule (II) sont notamment aisément accessibles à l'homme du métier, lorsque A représente un chaînon éthylène, par réduction d'un composé de formule (II/a) : 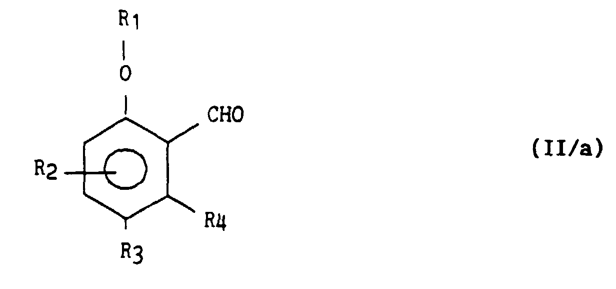 dans laquelle R₁, R₂, R₃ et R₄ sont tels que définis dans la formule (I), pour obtenir un composé de formule (II/b) : 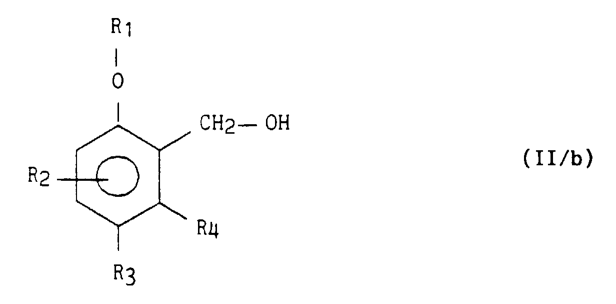 dans laquelle R₁, R₂, R₃, et R₄ sont tels que définis précédemment,
qui est ensuite soumis à un agent d'halogénation pour obtenir un composé de formule (II/c) : 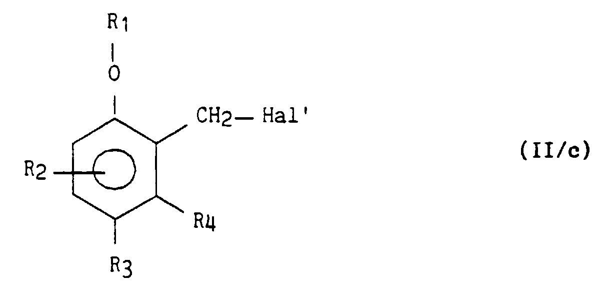 dans laquelle R₁, R₂, R₃, et R₄ sont tels que définis précédemment et Hal' représente un atome d'halogène,
composé de formule (II/c) qui est mis en réaction avec du cyanure de potassium afin d'obtenir le composé de formule (II/d) : 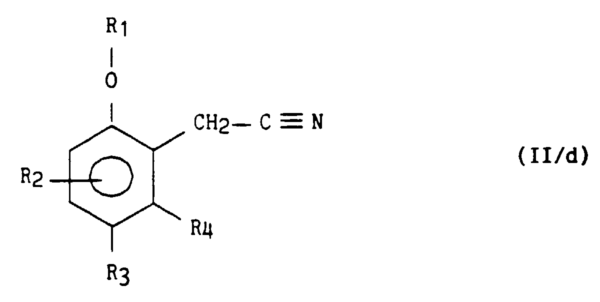 dans laquelle R₁, R₂, R₃ et R₄ sont tels que définis précédemment,
qui donne, après hydrogénation, le composé de formule (II/e) : 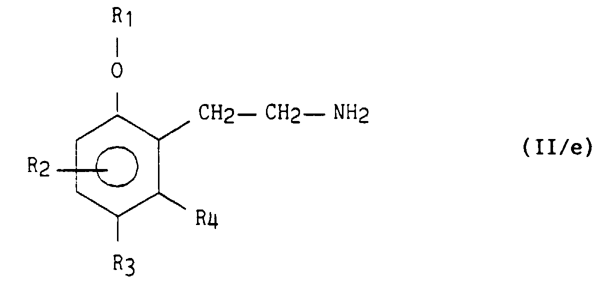 dans laquelle R₁, R₂, R₃, et R₄ sont tels que définis précédemment,
composé de formule (II/e) qui peut être, le cas échéant, mis en réaction avec un composé de formule (V)

Hal''- R₅' (V)

dans laquelle R₅' représente un alkyle linéaire ou ramifié de 1 à 6 atomes de carbone et Hal'' représente un atome d'halogène,
pour obtenir le composé de formule (II/e') : 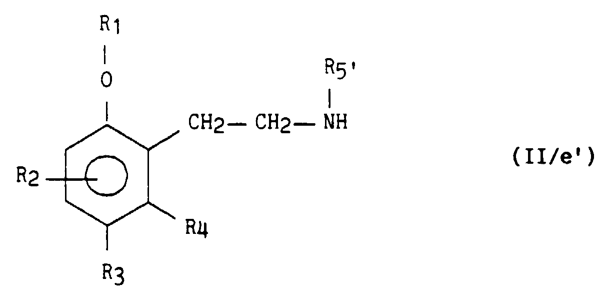 dans laquelle R₁, R₂, R₃, R₄, et R₅' sont tels que définis précédemment,
les composés de formule (II/e) et (II/e') formant l'ensemble des composés de formule (II),
les composés de formule (II) pouvant être :
- purifiés suivant une ou plusieurs méthodes de purification choisies parmi la cristallisation, la chromatographie sur colonne de silice, la chromatographie en phase gazeuse, l'extraction, la filtration, et le passage sur charbon ou résine,
- séparés, le cas échéant, sous forme pure ou sous forme de mélange, en leurs éventuels isomères optiques,
- ou salifiés par un acide ou une base pharmaceutiquement acceptable.

Les composés de formule (II/e) tels que définis précédemment sont également aisémment accessibles à l'homme du métier par réaction d'un composé de formule (II/a) : 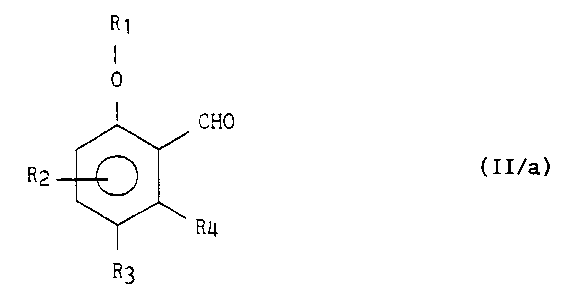 dans laquelle R₁, R₂, R₃ et R₄ sont tels que définis dans la formule (I) avec le nitrométhane afin d'obtenir un composé de formule (II/f) : 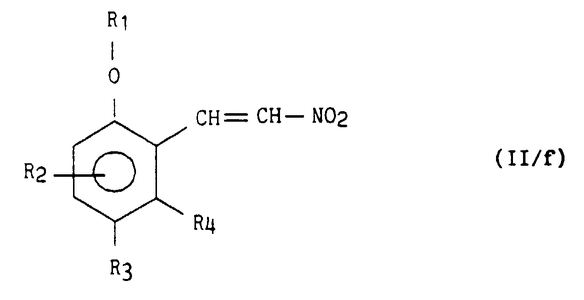 dans laquelle R₁, R₂, R₃ et R₄ sont tels que définis précédemment, qui est ensuite réduit pour obtenir le composé de formule (II/e) correspondant.

Les composés de formule (II) dans lesquels A représente un chaînon éthylène substitué par un alkyle inférieur peuvent être obtenus par l'homme du métier par réaction d'un composé de formule (II/a) : 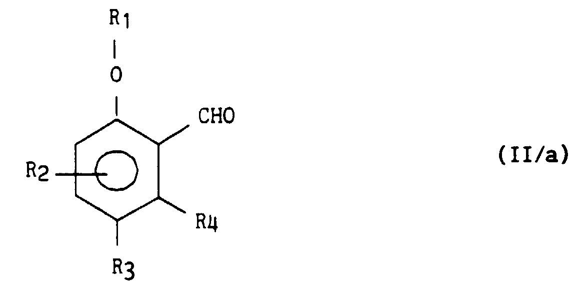 dans laquelle R₁, R₂, R₃ et R₄ sont tels que définis dans la formule (I) avec un composé nitré de formule (II/g) :

A' - NO₂ (II/g)

dans laquelle A' représente un groupement méthyle substitué par un alkyle inférieur afin d'obtenir un composé de formule (II/f') : 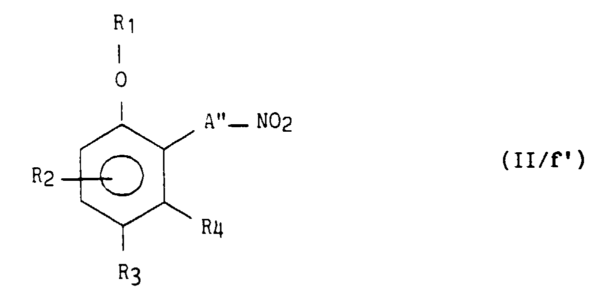 dans laquelle R₁, R₂, R₃ et R₄ sont tels que définis précédemment et A'' représente un chaînon éthylène substitué par un alkyle inférieur,
composé de formule (II/f') qui est soumis à une étape de réduction et éventuellement alkylé par réaction avec un composé de formule (V) :

Hal'''-R₅' (V)

dans laquelle R₅' représente un alkyle linéaire ou ramifié de 1 à 6 atomes de carbone et Hal''' représente un atome d'halogène, afin d'obtenir le composé de formule (II) correspondant.

La demanderesse a découvert que les composés de l'invention possédaient une remarquable activité sur le système mélatoninergique. Ils présentent notamment une très haute affinité pour les récepteurs de la mélatonine.

Cette capacité de liaison très importante est mis en évidence dans l'exemple B (Etude de liaison aux récepteurs de la mélatonine) de la présente demande.
L'intensité de cette propriété pharmacologique des composés de l'invention s'est avérée surprenante puisqu'elle ne se retrouve pas pour les composés dans lesquels le groupement hydroxyle ou alcoxy substitue en position méta ou para le noyau benzène portant la chaîne alkylamidique.

De par leur action sur les récepteurs de la mélatonine, les composés de l'invention sont donc de nouveaux candidats pour le traitement des troubles du système mélatoninergique.

Les pathologies où intervient la mélatonine sont notamment les troubles du sommeil, la dépression, la maladie de Parkinson, la maladie d'Alzheimer, le cancer, les troubles dus aux décaiage horaire, les troubles immunitaires, la migraine, le diabète, et le stress. La mélatonine est également impliquée dans un mécanisme d'inhibition de l'ovulation.

L'invention s'étend aussi aux compositions pharmaceutiques contenant comme principe actif au moins un des composés de formule (I) ou un de ses sels d'addition avec un acide ou une base pharmaceutiquement acceptable, en association avec un ou plusieurs excipients pharmaceutiquement acceptables.

Parmi les compositions selon l'invention, on pourra citer, à titre d'exemples et de façon non limitative, celles qui conviennent pour l'administration orale, parentérale, oculaire, per ou trans-cutanée, nasale, rectale, perlinguale, ou respiratoire, et notamment les préparations injectables, les aérosols, les gouttes oculaires ou nasales, les comprimés, les comprimés sublinguaux, les gélules, les capsules, les tablettes, les glossettes, les suppositoires, les crèmes, les pommades, et les gels.

Les préparations ainsi obtenues se présentent généralement sous forme dosée et peuvent contenir selon les affections traitées, l'âge, et le sexe du malade, de 0,01 à 100 mg en prises de une à trois fois par jour, plus particulièrement de 0,1 à 100 mg, par exemple de 0,1 à 10 mg.

Les exemples qui suivent illustrent l'invention et ne la limitent en aucune façon. La structure des composés est vérifiée par résonance magnétique nucléaire.

### PREPARATION 1 : 2-(2-METHOXY-NAPHT-1-YL)-ETHYLAMINE

### Premier procédé :

### STADE A : (2-METHOXY-NAPHT-1-YL)-METHANOL

Dans un bicol de 250 cm³, on place sous argon 40 cm³ de tétrahydrofurane anhydre (THF). On additionne 3,1 g d'hydrure de lithium et d'aluminium. A ce mélange, sous agitation, est ajoutée une solution de 5 g de (2-méthoxy-napht-1-yl)-carbaldéhyde dans 30 cm3 de tétrahydrofurane anhydre. On laisse ensuite évoluer à température ambiante pendant 16 h. L'hydrolyse est effectuée selon le procédé suivant :
- on additionne 3,1 cm3 d'eau au mélange et on laisse 5 min sous agitation,
- on ajoute alors 3,1 cm3 de solution d'hydroxyde de sodium à 15 %, on laisse de nouveau 5 min en agitation,
- puis, on additionne 9,3 cm3 d'eau jusqu'à l'obtention d'un mélange pateux blanchâtre.
- Après incorporation de sulfate de sodium et une nouvelle agitation pendant quelques minutes, le mélange est filtré et le filtrat concentré au rotavapor.
- On obtient ainsi des cristaux blancs de l'alcool recherché représentant une masse de 4,87 g (Rendement : 96 %).

Le composé est recristallisé à chaud dans un mélange éther isopropylique-éthanol, pour donner 3,65 g de cristaux blancs de (2-méthoxy-napht-1-yl)-méthanol.
Point de fusion : 100,9°C ± 0,2
RMN¹H (CDCl₃, 200 MHz)
. 2,01 ppm (1H (OH), s.e.)
. 3,90 ppm (3H (CH₃O), s.)
. 5,10 ppm (2H (CH₂O), s.)
. 7,17 - 8,06 ppm (6H (aromatique), m.)

### STADE B : 1-CHLOROMETHYL-2-METHOXY-NAPHTALENE

Dans un bicol de 100 cm³, on place 2,21 g du composé obtenu au stade A. Le ballon est mis sous argon. On incorpore 17,6 cm³ de toluène (1,5 équivalent) puis 0,94 cm³ de pyridine (1 équivalent). Le ballon est plongé dans un bain de glace. Par une ampoule à brome isobare, on additionne alors une solution de 2 cm³ de chlorure de thionyle dans 12 cm³ de toluène. Un précipité blanc se forme. Après addition, le bain de glace est oté. On laisse évoluer à température ambiante pendant 15 h.
Ce mélange est versé dans de la glace ; on laisse le milieu réactionnel sous agitation pendant 45 min. Après séparation des phases aqueuse et organique, cette dernière est lavée une fois à l'eau, une fois par une solution saturée d'hydrogénocarbonate de sodium puis une fois par une solution saturée en chlorure de sodium. Après séchage sur sulfate de magnésium et concentration du filtrat, on isole un produit cristallin jaune d'une masse de 2,24 g, correspondant au 1-chlorométhyl-2-méthoxynaphtalène (Rendement : 92,3 %)
Ces cristaux peuvent être recristallisés à chaud dans l'éher éthylique ; le 1-chlorométhyl-2-méthoxy-naphtalène est un solide blanc cristallin.
Point de fusion : 122,7°C ± 0,2
RMN¹H (CDCl₃, 200 MHz)
. 3,92 ppm (3H (OCH₃), s.)
. 5,10 ppm (2H (CH₂Cl), s.)
. 7,18 ppm (1H (arom.), s. J = 8,9 Hz)
. 7,27 - 7,34 ppm (1H (aromatique), m.)
. 7,45 - 7,53 ppm (1H (aromatique), m.)
. 7,73 ppm (1H (aromatique), d. J = 8,9 Hz)
. 7,77 ppm (1H (aromatique), d. J = 8,9 Hz)
. 7,96 ppm (1H (aromatique), d. J = 8,9 Hz)

### STADE C : 1-CYANOMETHYL-2-METHOXY-NAPHTALENE

La réaction met en jeu 1,14 g du composé obtenu au stade B, 0,718 g de cyanure de potassium (2 équivalents), et une petite spatule d'iodure de potassium, le tout dans 12 cm³ de diméthylsulfoxyde anhydre (2 équivalents). Elle est réalisée sous argon, à 120-140°C, et dure 8 h. Par traitement post-réactionnel on obtient des cristaux de couleur rouille qui sont recristallisés à chaud dans un mélange hexane-acétate d'éthyle.
On isole ainsi 0,665 g de cristaux beiges de 1-cyanométhyl-2-méthoxynaphtalène (Rendement : 61 %)
Point de fusion : 95,4°C ± 0,2
RMN¹H (CDCl₃, 200 MHz)
. 3,94 ppm (3H (CH₃O), s.)
. 4,05 ppm (2H (CH₂CN), s.)
. 7,18 - 7,83 ppm (6H (aromatique), m.)

### STADE D : 2-(2-METHOXY-NAPHT-1-YL)-ETHYLAMINE

La réaction est effectuée sur 0,6 g du composé obtenu au stade précédent (3 mmoles), dans 10 cm³ d'éthanol absolu (3 équivalents) et 1 cm³ d'hydroxyde d'ammonium.
Le composé est hydrogéné à pression atmosphérique et à température ambiante sur du nickel de Raney. On laisse évoluer 23 heures. Le catalyseur est éliminé par filtration sur couche de célite et rincé par de l'éthanol absolu. Le filtrat est concentré au rotavapor. Puis on forme le chlorhydrate de l'amine : Le mélange est dilué dans du méthanol, on ajoute du méthanol chlorhydrique, et le chlorhydrate est précipité par addition d'éther isopropylique.
On isole ainsi 0,468 g de cristaux jaunes de chlorhydrate de la 2-(2-méthoxy-napht-1-yl)-éthylamine (Rendement : 64,5 %)
Point de fusion : > 180°C
RMN¹H (CD₃OD, 200 MHz)
. 3,30 - 3,37 ppm (2H (CH₂-), m.)
. 3,62 - 3,69 ppm (2H (CH₂N), m.)
. 4,19 ppm (3H (CH₃O), s.)
. 7,55 - 7,76 ppm (3H (aromatique), m.)
. 8,01 - 8,20 ppm (3H (aromatique), m.)

### Second procédé :

### STADE A' : 1-NITRO-2-(2-METHOXY-NAPHT-1-YL)ETHYLENE

Dans un ballon bicol de 50 cm³ muni d'un réfrigérant, on introduit 1,52 g de 2-méthoxy-naphtaldéhyde (8,16 mmol), 0,4 g d'acétate d'ammonium (5,19 mmol) et 20 cm³ de nitrométhane (96 %). En agitant, on chauffe à reflux pendant 1h30 min. Le mélange réactionnel devient jaune. On évapore le nitrométhane à l'évaporateur rotatif puis on reprend le résidu avec du dichlorométhane. L'hydrolyse de l'acétate d'ammonium est réalisée en versant le mélange précédent dans de l'eau. La phase aqueuse est lavée deux fois avec CH₂Cl₂, la phase organique une fois avec de l'eau. Cette dernière est séchée par MgSO₄, et le solvant est éliminé à l'évaporateur rotatif. On obtient alors 1,77 g de cristaux jaunes (soit 7,73 mmol du produit attendu), ce qui correspond à un rendement de 95 %.
Point de fusion : 138,6 ± 0,4°C
RMN 1H (CDCl₃, 200 MHz)
. 4,04 ppm (3H (MeO), s)
. 8,82-8,76 ppm (1H (CHNO₂), d, J = 13,35 H₃)
. 8,13-8,06 ppm (1H (naph-CH), d, J = 13,42 H₃)
. 7,23-8,14 ppm (6H (arom), multiplet constitué de 2 triplets dédoublés : 7,60-7,56-7,52 ppm, J=7,15 Hz, 7,41-7,37-7,34 ppm J=7,2 Hz et de 4 doublets : 8,14-8,09 ppm J=8,55 Hz, 7,94-7,90 ppm J=9,18 Hz, 7,78-7,74 ppm J=8Hz, 7,28-7,23 ppm J=9,7 Hz)
Chromatographie couche mince :
R_{f} = 0,93 (CH₂Cl₂)

### STADE B' : 2-(2-METHOXY-NAPHTYL)ETHYLAMINE

Dans un ballon tricol de 250 cm³ muni d'un réfrigérant et d'une ampoule à brome de 100 cm³, on introduit sous atmosphère inerte 50 cm³ de THF anhydre puis, en refroidissant le ballon avec de la glace et en agitant le milieu réactionnel, 1,74 g (45,82 mmol) de LiAlH4. Dans les mêmes conditions, on additionne ensuite en 1 h un mélange constitué de 2,05 g (8,94 mmol) de composé obtenu au stade précédent et de 75 cm³ de THF. Après introduction des réactifs, le mélange est chauffé pendant environ 24 h à une température comprise entre 30 et 40°C. L'hydrolyse de l'hydrure excédentaire est effectuée en refroidissant de nouveau le ballon par de la glace et en ajoutant 1,6 cm³ d'eau, 1,6 cm³ de NaOH 15 %, 37 cm³ d'éther et 4,7 cm3 d'eau. Après filtration, le mélange est séché par du carbonate de sodium anhydre, concentré à l'évaporateur rotatif et purifié par chromatographie sur une colonne de silice (éluants : acétate d'éthyle, Rf = 0 puis un mélange constitué de 15 % de méthanol, de 10 % de triéthylamine et de 75 % d'acétate d'éthyle, Rf = 0,28). On obtient finalement 1,42 g (7,06 mmol) de 2-(2-méthoxy-naphtyl)-éthylamine (huile visqueuse jaune orangée), soit un rendement après purification de 78,9 %.

### PREPARATIONS 2 A 7 :

En procédant comme dans la préparation 1 mais en remplaçant au stade le (2-méthoxy-napht-1-yl)-carbaldéhyde par :
- le (2,5,6-triméthoxy-napht-1-yl)-carbaldéhyde (Eur. J. Med. Chem. 1980, 19 (3) pp 249-253), on obtient :

### PREPARATION 2 : LA 2-(2,5,6-TRIMETHOXY-NAPHT-1-YL)-ETHYLAMINE

- le (6-hydroxy-2-méthoxy-napht-1-yl)-carbaldéhyde (Eur. J. Med. Chem. 1983, 18 (2), pp 169-174), on obtient :

### PREPARATION 3 : LA 2-(6-HYDROXY-2-METHOXY-NAPHT-1-YL)-ETHYLAMINE

- le (2,6-dipropoxy-napht-1-yl)-carbaldéhyde (J. Org. Chem. 1981, 46 (9), pp 1832-1835), on obtient :

### PREPARATION 4 : LA 2-(2,6-DIPROPOXY-NAPHT-1-YL)-ETHYLAMINE

- le (5,6,7,8-tétrahydro-4-méthyl-2-méthoxy-napht-1-yl)-carbaldéhyde (Aust. J. Chem. 1981, 34 (2), pp 459-464), on obtient :

### PREPARATION 5 : LA 2-(5,6,7,8-TETRAHYDRO-4-METHYL-2-METHOXY-NAPHT-1-YL)-ETHYLAMINE

- le 5-formyl-6-méthoxy-1,4-benzodioxane (J. Heterocycl. Chem. 1989, 26 (1), pp 193-197), on obtient :

### PREPARATION 6 : LA 2-(6-METHOXY-1,4-BENZODIOXAN-5-YL)-ETHYLAMINE

- le 7-formyl-6-méthoxy-3-méthyl-benzofurane (Bull. Soc. Chim. Fr. 1975, 11-12, Pt.2, pp 2763-2766), on obtient :

### PREPARATION 7 : LA 2-(6-METHOXY-3-METHYL-BENZOFURAN-7-YL)-ETHYLAMINE

### PREPARATION 8 : 2-(5-BROMO-2-METHOXY-PHENYL)-ETHYLAMINE

### STADE A : (5-BROMO-2-METHOXY-PHENYL)-METHANOL

Dans un bicol de 250 cm³, on place 30 cm³ de tétrahydrofurane anhydre. Le ballon étant glacé, on additionne 2,66 g d'hydrure de lithium et d'aluminium. Par une ampoule à brome isobare, on additionne 5,014 g de 5-bromo-2-méthoxy-benzaldéhyde en solution dans 30 cm³ de tétrahydrofurane anhydre (le montage est sous atmosphère d'argon). L'ampoule à brome est ensuite rincée par 10 cm³ de tétrahydrofurane anhydre et le mélange est laissé à température ambiante 5 h sous forte agitation. L'hydrolyse est réalisée ainsi :
- addition de 2,7 cm³ d'eau, agitation de ce mélange pendant 5 min,
- addition de 2,7 cm³ de solution d'hydroxyde de sodium 15 %, agitation pendant 5 min,
- puis addition de 3 fois 2,7 cm³ d'eau jusqu'à obtenir une masse blanche.
- On incorpore alors du sulfate de sodium afin de sécher l'ensemble.
- Après filtration et concentration du filtrat, on obtient un solide blanc de 4,4 g dont l'analyse indique qu'il s'agit de l'alcool désiré.
RMN¹H (CDCl₃, 200 MHz)
. 2,38 ppm (1H (OH), s.e.)
. 3,75 ppm (3H (CH₃O), s.)
. 4,55 ppm (2H (CH₂O), s. e.)
. 6,66 ppm (1H (aromatique), d. J = 8,6 Hz)
. 7,17 - 7,34 ppm (2H (aromatique), m.)

### STADE B : CHLORURE DE 5-BROMO-2-METHOXY-BENZYLE

Dans un bicol de 100 cm³, on place 4,023 g du composé obtenu au stade A. Il est dissous dans 28 cm³ de toluène anhydre. Le tout est mis sous argon. On introduit à la seringue 1,5 cm³ de pyridine.

A ce mélange sous agitation et glacé, on additionne par une ampoule à brome isobare 3,2 cm³ de chlorure de thionyle dilué dans 18 cm³ de toluène anhydre. Après addition, on ôte le bain de glace. On laisse la réaction évoluer à température ambiante pendant 18 h. Le mélange est hydrolysé en le versant dans de la glace. On laisse le tout sous forte agitation pendant 45 min. Les phases organique et aqueuse sont séparées. La phase organique est lavée à l'eau, puis par une solution aqueuse saturée en hydrogénocarbonate de sodium et par une solution aqueuse saturée en chlorure de sodium. Après séchage sur sulfate de magnésium et concentration du filtrat on isole un liquide orange qui est chromatographié sur silice (éluant : éther/pentane : 20/80 - 40/60). On recueille ainsi un liquide incolore, qui cristallise à froid, de masse 4,17 g.
RMN¹H (CDCl₃, 200 MHz)
- 3,78 ppm (3H (CH₃O), s.)
- 4,51 ppm (2H (CH₂Cl), s.)
- 6,69 ppm (1H (aromatique), d. J = 8,6 Hz)
- 7,24 - 7,41 ppm (2H (arom.), m.)

### STADE C : 5-BROMO-1-CYANOMETHYL-2-METHOXYBENZENE

On ajoute, à 4,03 g du composé obtenu précédemment, 2,23 g de cyanure de potassium, une petite spatule d'iodure de potassium, dans 35 cm³ de diméthylsulfoxide anhydre.
Ce mélange sous argon est porté à 120-140°C pendant 5 h.
Après traitement post-réactionnel, on obtient un liquide de couleur rouille qui est chromatographié sur silice (éluant : éther/pentane : 30/70 - 100/0).
Afin d'obtenir le composé pur, on recristallise la première fraction à chaud dans un mélange hexane-acétate d'éthyle.
On isole ainsi 1,35 g de cristaux jaunes de 5-bromo-1-cyanométhyl-2-méthoxybenzène, dont l'analyse confirme la pureté.
Point de fusion : 60,7°C ± 0,2
RMN¹H (CDCl₃, 200 MHz)
. 3,58 ppm (2H (CH₂CN), s.)
. 3,78 ppm (3H (CH₃O), s.)
. 6,70 ppm (1H (aromatique), d. J = 8,7 Hz)
. 7,34 ppm (1H (aromatique), d.d. J = 2,3 Hz, J = 8,7 Hz)

### STADE D : 2-(5-BROMO-2-METHOXY-PHENYL)-ETHYLAMINE

En procédant comme dans le stade D de la préparation 1 mais en remplaçant le 1-cyanométhyl-2-méthoxy-naphtalène par le 5-bromo-1-cyanométhyl-2-méthoxybenzène obtenu au stade précédent, on obtient la 2-(5-bromo-2-méthoxy-phényl)-éthylamine.

### PREPARATION 9 : 1-METHYL-2-(2-METHOXY-NAPHT-1-YL)ETHYLAMINE

### STADE A : 2-NITRO-1-(2-METHOXY-NAPHT-1-YL)PROPENE

Dans un ballon, on introduit 2,13 g (11,20.10-3 mol) de naphtaldéhyde, 30 cm³ de nitroéthane et 0,54 g (7.10-3 mol) d'acétate d'ammonium. On agite le mélange et on chauffe à reflux pendant deux heures. Puis on évapore le nitroéthane sous pression réduite, on reprend le résidu par CH₂Cl₂ et on ajoute de l'eau pour hydrolyser l'acétate. Après séparation des deux phases, la phase organique est lavée avec de l'eau, les phases aqueuses par CH₂Cl₂ (2 fois). La phase organique est alors séchée sur MgSO₄ et concentrée sous pression réduite. On obtient alors 2,59 g (10,64.10-3 mol) du composé nitroéthylénique attendu (solide jaune), soit un rendement de 95 %.
Point de fusion : 72 ± 3°C
RMN ¹H(200 MHz, CDCl₃) δ(ppm) :
. composé Z : 2,1 (s, 3H, CH₃) ; 3,97 (s, 3H, MeO) ; 7,23/7,96 (n, 6H, aromatiques) ; 8,33 (s, 1H, H éthylénique (68 %)
. Composé E : 2,52 (s, 3H, CH₃) ; 3,89 (s, 3H, MeO) ; 6,92 (s, 1H, H éthylénique) ; 7,23/7,96 (n, 6H, aromatiques) (32 %)

### STADE B : 1-METHYL-2-(2-METHOXY-NAPHT-1-YL)ETHYLAMINE

Dans un tricol de 250 cm³ sous atmosphère inerte, on introduit 50 cm³ de THF anhydre, on agite et on refroidit le ballon par de la glace. On introduit 1,45 g (38,19.10-3 mol) de LiAlH₄ par petites fractions, puis goutte à goutte un mélange constitué de 60 cm³ de THF anhydre et de 2,09 g (8,59.10-3 mol) du composé nitro éthylénique. On enlève alors le bain de glace et après retour à la température ambiante, on chauffe le mélange à 40°C pendant 24 h. L'hydrolyse de l'hydrure excédentaire est réalisée comme suit : le mélange étant refroidit par de la glace, on ajoute 1,5 cm³ d'eau, 1,5 cm3 de soude à 15 %, 30 cm³ d'éther, et 4,5 cm³ d'eau. On filtre puis on séche le filtrat sur MgSO₄, et on évapore à sec. Le résidu obtenu est purifié par chromatographie sur colonne de silice (éluant acétate d'éthyle puis mélange acétate d'éthyle méthanol/triéthylamine 15/3/2). On isole finalement 1,09 g (5,06.10⁻³ mol) de l'amine attendue, soit un rendement de 59 %.

### PREPARATION 10 : 2-(2-METHOXY-5-METHYL-PHENYL)ETHYLAMINE

### STADE A : 2-METHOXY-5-METHYL-BENZOATE DE METHYLE

Dans un bicol de 250 cm³, on introduit 5,07 g (33,32.10⁻³ mol) d'acide 5-méthyl salicylique et 22,33 g (161,6.10⁻³ mol) de carbonate de potassium anhydre. Sous atmosphère inerte, 175 cm³ d'acétone anhydre sont ajoutés. Le mélange réactionnel étant agité, on ajoute à la seringue 7,25 cm³ (76,62.10⁻³ mol) de diméthyl sulfate, puis on porte à reflux durant 6 h. L'hydrolyse du diméthyl sulfate est effectuée en ajoutant 5 cm³ d'eau. Après filtration et évaporation du solvant, le résidu est repris par du dichlorométhane, séché sur MgSO₄ puis évaporé à sec. Le produit brut est purifié par chromatographie sur colonne de silice (éluant : CH₂Cl₂) pour donner 5,47 g (30,36.10⁻³ mol) de l'ester attendu, soit un rendement de 91 %.
Température d'ébullition (12 mmHg) : 128-132°C

### STADE B : (2-METHOXY-5-METHYL-PHENYL)METHANOL

Dans un tricol de 500 cm³ contenant 100 cm³ de THF anhydre et sous atmosphère inerte, on introduit 1,56 g (41,08.10⁻³ mol) de LiAlH₄ en refroidissant le ballon par un bain de glace. Puis un mélange constitué de 4,99 g (27,69.10⁻³ mol) de l'ester précédent et de 150 cm³ de THF anhydre est introduit goutte à goutte en une heure, le ballon étant maintenu à 0°C.
Après retour à la température ambiante, le milieu réactionnel est chauffé à reflux durant 20 h. L'hydrolyse de l'hydrure excédentaire est effectuée en refroidissant le ballon par de la glace et en ajoutant lentement 1,3 cm³ d'eau puis 1,3 cm³ de soude 15 %, 40 cm³ d'éther et 5 cm³ d'eau. Après filtration, séchage du filtrat par MgSO₄ et évaporation à sec de celui-ci, le résidu est purifié par distillation sous vide pour donner 3,68 g (24,18.10⁻³ mol) de l'alcool attendu, soit un rendement de 87 %.
Point d'ébullition (12 mmHg) : 130-132°C

### STADE C : CHLORURE DU 2-METHOXY-5-METHYL-BENZYLE

Dans un tricol de 100 cm³, on introduit 3,23 g (21,22.10⁻³ mol) de l'alcool benzylique et 50 cm³ de toluène anhydre. Sous argon, en agitant et refroidissant le mélange réactionnel, on introduit 1,70 cm³ (21,10.10⁻³ mol) de pyridine séchée sur potasse, puis 3,5 cm³ (47,98.10⁻³ mol) de chlorure de thionyle. Il se forme un précipité blanc et le chlorure d'hydrogène se dégage. Après introduction des réactifs, on laisse évoluer à température ambiante durant environ 24 h. Le mélange est ensuite versé dans un bain de glace et agité durant 1 h. La phase organique est récupérée, lavée par de l'eau, une solution saturée de bicarbonate de sodium, une solution saturée de chlorure de sodium, et enfin séchée sur MgSO₄. Après évaporation à sec, le résidu est purifié par distillation sous vide et on obtient 2,5 g (14,65.10⁻³ mol) du chlorure attendu, soit un rendement de 69 %.
Température d'ébullition : (13 mmHg) : 117-120°C

### STADE D : 1-CYANOMETHYL-2-METHOXY-5-METHYL BENZENE

Dans un tricol de 250 cm³, on introduit 2,44 g (14,30.10⁻³ mol) du chlorure de benzyle obtenu au stade précédent et 50 cm³ de diméthylsulfoxide (DMSO). Le mélange étant agité et sous atmosphère inerte, on ajoute une spatule d'iodure de potassium et 1,85 g (28,41.10⁻³ mol) de cyanure de potassium. On laisse évoluer à température ambiante durant 20 h. Après évaporation du DMSO sous vide, on reprend le résidu par CH₂Cl₂, et on hydrolyse KCN et KI en ajoutant de l'eau. La phase aqueuse est lavée par du dichlorométhane, et les phases organiques jointes par de l'eau. Ces dernières sont ensuite séchées sur MgSO₄ et évaporées à sec. Le produit brut obtenu est purifié par distillation sous pression réduite et on obtient 2,11 g (13,09.10⁻³ mol) du nitrile attendu soit un rendement de 91 %.
Température d'ébullition (16 mmHg) = 136-140°C

### STADE E : 2-(2-METHOXY-5-METHYL-PHENYL)ETHYLAMINE

Dans un tricol de 100 cm³, on introduit 1,7 g (10,54.10⁻³ mol) de nitrile, 38 cm³ d'éthanol 95.96 % et 3,8 cm³ d'ammoniaque.
Sous atmosphère inerte, on introduit une pointe de spatule de Nickel de Raney. Après avoir fait le vide dans l'ensemble du montage, on instaure dans ce dernier une atmosphère d'hydrogène et on agite fortement le milieu réactionnel. On chauffe ensuite à 40-50°C durant environ 48 h. Après refroidissement, on filtre le mélange sur célite et on lave par de l'éthanol. Le filtrat est évaporé à sec, repris par de l'éther et évaporé de nouveau. Le produit brut obtenu est purifié par passage au chlorhydrate de l'amine obtenue. On obtient un produit solide jaune verdâtre qu'il est nécessaire de recristalliser dans un mélange éthanol-éther. On obtient alors 1,07 g (5,31.10⁻³ mol) de chlorhydrate blanc.
Point de fusion : 170 ± 3°C

| Analyse élémentaire : | |
|---|---|
| C théo = 59,55 % | C exp = 59,39 % |
| H théo = 7,99 % | H exp = 7.85 % |
| N théo = 6,94 % | N exp = 6,78 % |
| Cl théo = 17,58 % | Cl exp = 17,54 % |

### PREPARATION 11 : 2-(5-ETHYL-2-METHOXY-PHENYL)ETHYLAMINE

Dans un tricol de 50 cm³ sous atmosphère inerte, on introduit 20 cm³ de THF anhydre, on agite et on refroidit le solvant par de la glace. Puis on introduit 0,43 g (11,32.10⁻³ mol) de LiAlH₄ par petites fractions. Puis, on introduit goutte à goutte 0,51 g (2,45.10⁻³ mol) du 1-nitro-2-(5-éthyl-2-méthoxy-phényl)éthylène préparé selon les méthodes de synthèse connues de l'homme du métier.
Après introduction, on chauffe le milieu réactionnel à 40°C durant 18 h. Après refroidissement, on hydrolyse l'hydrure excédentaire en ajoutant au mélange refroidi par de la glace, 0,5 cm³ d'eau, 0,5 cm³ de NaOH 15 %, 15 cm³ d'éther, et 1,5 cm³ d'eau. Après filtration, le filtrat est séché sur MgSO₄ et évaporé à sec et le résidu est purifié par chromatographie sur colonne de silice (éluant : acétate d'éthyle puis acétate d'éthyle/MeOH/Et₃N : 15/3/2). L'amine obtenue est solubilisée dans CH₂Cl₂ et on ajoute alors 2 équivalents (eq.) d'éther chlorhydrique. Après évaporation des solvants, on obtient un précipité beige, qu'il est nécessaire de recristalliser dans un mélange éthanol-éther. On obtient finalement 150 mg (6,95.10-3 mol) soit un rendement final de 28 % du chlorhydrate de l'amine attendue.
Point de fusion : 173 ± 3°C

### EXEMPLE 1 : N-[2-(2-METHOXY-NAPHT-1-YL)ETHYL]-ACETAMIDE

Dans un bicol de 50 cm³, on place 0,3 g (1,2 mmoles) du chlorhydrate de 2-(2-méthoxy-napht-1-yl)éthylamine (préparation 1).
On ajoute 10 cm3 d'eau distillée jusqu'à dissolution des cristaux, puis 0,1 g d'acétate de sodium (13 équivalents). A ce mélange sous agitation est additionné 3 cm3 d'anhydride acétique (25 équivalents). La solution devient laiteuse, un léger échauffement se produit. puis elle redevient limpide. Après 25 min de réaction, le mélange est extrait au dichlorométhane. Les phases organiques, lavées par une solution aqueuse saturée en hydrogénocarbonate de sodium, puis à l'eau sont séchées sur sulfate de magnésium. Le filtrat concentré donne des cristaux jaunes pâles qui sont directement recristallisés à chaud dans un mélange hexane-acétate d'éthyle.
On obtient ainsi 0.235 g de cristaux blancs de N-[2-(2-méthoxy-napht-1-yl)-acétamide.
Rendement : 76,8 %
Point de fusion : 138,7°C
RMN¹H (CDCl₃, 200 MHz)
. 3,22 - 3,29 ppm (2H (CH₂N), m.)
. 3,43 - 3,52 ppm (2H (CH₂O), m.)
. 3,90 ppm (3H (CH₃O), s.)
. 5,69 ppm (1H (NH), s. e.)
. 7,19 - 7,31 ppm (2H (aromatique), m.)
. 7,39 - 7,47 ppm (1H (aromatique), m.)
. 7,68 - 7,74 ppm (2H (aromatique), m.)
. 7,94 ppm (1H (arom.), d. J = 8,6 Hz)

### EXEMPLE 2 : N-[2-(2-METHOXY-NAPHT-1-YL)ETHYL]-PROPIONAMIDE

### Premier procédé :

Dans un bicol de 25 cm³, on place 0,22 g de 2-(2-méthoxy-napht-1-yl)-éthylamine. Le composé est dilué par 5,3 cm³ de dichlorométhane anhydre (3 équivalents). Après avoir mis le mélange sous argon et glacé le ballon, on additionne au mélange 0,250 cm³ de triéthylamine, puis lentement 0,160 cm³ de chlorure de propanoyle (1 équivalent chacun). Après 30 min de réaction à température ambiante, par traitement post-réactionnel, nous obtenons un liquide brun qui est chromatographié sur colonne de silice (éluant : CH₂Cl₂/méthane : 0,2 %). Le produit isolé cristallise à température ambiante. On le fait recristalliser par dissolution à chaud dans un mélange hexane-acétate d'éthyle.
On obtient ainsi 0,193 g de cristaux beiges de N-[2-(2-méthoxy-napht-1-yl)-éthyl]-propionamide.
Point de fusion : 100,2°C ± 0,2
RMN¹H (CDCl₃, 200 MHz)
. 1,01 ppm (3H (CH₃), t. J = 7,6 Hz)
. 2,05 ppm (2H (CH₂), q. J = 7,6 Hz)
. 3,23 - 3,29 ppm (2H (CH₂), m.)
. 3,44 - 3,54 ppm (2H (CH₂N), m.)
. 3,90 ppm (3H (CH₃O), s.)
. 5,70 ppm (1H (NH), s.e.)
. 7,24 - 7,47 ppm (3H (aromatique), m.)
. 7,68 - 7,73 ppm (2H (aromatique), m.)
. 7,95 ppm (1H (arom.), d. J = 8,6 Hz)
Analyse élémentaire (C₁₆H₁₉NO₂)

| Analyse élémentaire (C₁₆H₁₉NO₂) | | |
|---|---|---|
| | calculé | trouvé |
| C | 74,68 % | 74,15 % |
| H | 7,44 % | 7,38 % |
| N | 5,44 % | 5,26 % |

### Second procédé :

Dans un bicol de 100 cm³ sous atmosphère inerte, on introduit 1,40 g (6,96 mmol) de 2-(2-méthoxy-naphtyl)-éthylamine que l'on solubilise dans 20 cm³ de dichlorométhane anhydre. Le mélange étant refroidi par un bain de glace et agité, on introduit 1,5 cm³ (10,4 mmol) de triéthylamine séchée sur potasse et 0,7 cm³ (8,056 mmol) de chlorure de propanoyle. Après introduction des réactifs, on enlève le bain de glace et on laisse évoluer la réaction à température ambiante pendant 30 min. L'hydrolyse de chlorure excédentaire est réalisée en versant le mélange réactionnel dans de l'eau. Après 3 extractions avec du dichlorométhane, la phase organique est séchée sur du sulfate de magnésium. Après évaporation du solvant et recristallisation à chaud du solide obtenu dans un mélange pentane/acétate d'éthyle, on obtient 0,70 g (2,72 mmol) de N-[2-(2-méthoxy-napht-1-yl)-éthyl]-propionamide (solide blanc) soit un rendement après purification de 39 %.

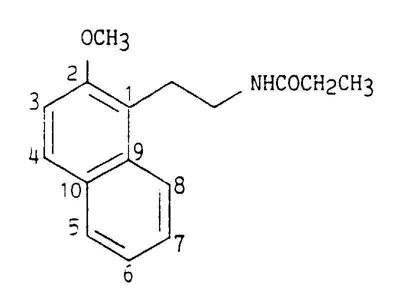

### EXEMPLE 3 : N-[2-(2-METHOXY-NAPHT-1-YL)ETHYL]-CYCLOPROPYLCARBOXAMIDE

En procédant selon l'exemple 2, mais en utilisant le chlorure de l'acide cyclopropane-carboxylique au lieu du chlorure de propanoyle, on obtient le composé du titre :
Point de fusion : 127,5°C ± 0,2
RMN¹H (CDCl₃, 200 MHz)
. 0,56 - 0,65 ppm (2H (CH₂ (cyclopropyl)), m.)
. 0,85 - 0,92 ppm (2H (CH₂ (cyclopropyl)), m.)
. 1,08 - 1,18 ppm (1H (CH (cyclopropyl)), m.)
. 3,23 - 3,30 ppm (2H (CH₂) m.)
. 3,44 - 3,54 ppm (2H (CH₂N) m.)
. 3,90 ppm (3H(CH₃-O), s.)
. 5,86 ppm (1H(NH), s.e.)

### EXEMPLE 4 : N-[2-(2-METHOXY-NAPHT-1-YL)ETHYL]-TRIFLUOROACETAMIDE

Dans un bicol de 25 cm³, on place 0,338 g de 2-(2-méthoxy-napht-1-yl)-éthylamine. On dilue par 5 cm³ de dichlorométhane anhydre (3 équivalents). Après avoir mis le ballon sous argon, on additionne 0,15 cm³ de pyridine stockée sur potasse (1,1 équivalent) et lentement, le ballon étant glacé, 0,24 cm³ d'anhydride trifluoroacétique (1 équivalent). La réaction dure 30 min à température ambiante. Par traitement post-réactionnel, on obtient 0,220 g de liquide jaune visqueux qui cristallise à température ambiante. Il est recristallisé par dissolution à chaud dans un mélange hexane-acétate d'éthyle.
On isole ainsi 52 mg de cristaux blancs correspondant au composé du titre :
Point de fusion 87,4°C ± 0,2
RMN¹H (CDCl₃, 200 MHz)
. 3,30 - 3,37 ppm (2H (CH₂), m.)
. 3,55 - 3,64 ppm (2H (CH₂N), m.)
. 3,91 ppm (3H (CH₃O), s.)
. 6.97 ppm (1H (NH), s.e.)
. 7,18 - 7,49 ppm (3H (arom.), m.)
. 7,72 - 7,87 ppm (3H (arom.), m.)
RMN ¹⁹F (CDCl₃, 188,3 MHz)
. 75,99 ppm (CF₃, s.)

### EXEMPLE 5 : N-[2-(2-METHOXY-NAPHTYL)ETHYL]-BUTYRAMIDE

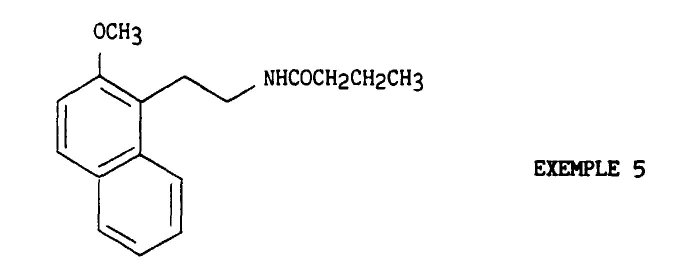

Dans un bicol de 100 cm³ sous atmosphère inerte, on introduit 0,45 g (2,23 mmol) de 2-(2-méthoxy-napht-1-yl)-éthylamine que l'on solubilise dans 20 cm³ de dichlorométhane anhydre. Le mélange étant refroidi par un bain de glace et agité, on introduit 0,47 cm³ (3,37 mmol) de triéthylamine séchée sur potasse puis 0,28 cm³ (2,69 mmol) de chlorure de butyryl. Après introduction des réactifs, on enlève le bain de glace et on laisse évoluer la réaction à température ambiante pendant 30 min. L'hydrolyse du chlorure excédentaire est réalisée en versant le mélange réactionnel dans de l'eau. Après trois extractions avec du dichlorométhane, la phase organique est séchée sur du sulfate de magnésium. Après évaporation du solvant et recristallisation à chaud du solide obtenu dans un mélnage pentane/acétate d'éthyle, on obtient 0,29 g (1,07 mmol) de N-[2-(2-méthoxy-naphtyl)-éthyl]- butyramide (solide blanc) soit un rendement après purification de 48 %.
Point de fusion : 76 ± 3°C

| Analyse élémentaire : | |
|---|---|
| C théo = 75,24 % | C exp = 75,14 % |
| H théo = 7,80 % | H exp = 7,84 % |
| N théo = 5,16 % | N exp = 5,16 % |

### EXEMPLE 6 : N-[2-(2-METHOXY-NAPHT-1-YL)ETHYL]PENTANAMIDE

Dans un bicol de 100 cm³ sous atmosphère inerte, on introduit 0,59 g (2,93 mmol) de 2-(2-méthoxy-napht-1-yl)-éthylamine que l'on solubilise dans 20 cm³ de dichlorométhane anhydre. Le mélange étant refroidi par un bain de glace et agité, on introduit 0,61 cm³ (4,37 mmol) de triéthylamine séchée sur potasse puis 0,42 cm³ (3,54 mmol) de chlorure de valéryle. Après introduction des réactifs, on enlève le bain de glace et on laisse évoluer la réaction à température ambiante pendant 30 min. L'hydrolyse du chlorure excédentaire est réalisée en versant le mélange réactionnel dans de l'eau. Après trois extractions avec du dichlorométhane, la phase organique est séchée sur du sulfate de magnésium. Après évaporation du solvant et recristallisation à chaud du solide obtenu dans un mélange pentane/acétate d'éthyle, on obtient 0,39 g (1,37 mmol) de N-[2-(2-méthoxy-naphtyl)éthyl]pentanamide (solide blanc) soit un rendement après purification de 47 %.
Point de fusion : 72 ± 3°C

### EXEMPLES 7 A 12 :

En procédant selon l'exemple 2, mais en remplaçant le chlorure de propanoyle par le chlorure d'acide approprié, on obtient les composés des exemples suivants :

### EXEMPLE 7 : N-[2-(2-METHOXY-NAPHT-1-YL)ETHYL]-HEXANAMIDE

### EXEMPLE 8 : N-[2-(2-METHOXY-NAPHT-1-YL)ETHYL]-HEPTANAMIDE

### EXEMPLE 9 : N-[2-(2-METHOXY-NAPHT-1-YL)ETHYL]-CROTONAMIDE

### EXEMPLE 10 : N-[2-(2-METHOXY-NAPHT-1-YL)ETHYL]-CYCLOBUTYLCARBOXAMIDE

### EXEMPLE 11 : N-[2-(2-METHOXY-NAPHT-1-YL)ETHYL]-4-CHLORO-BUTYRAMIDE

### EXEMPLE 12 : N-[2-(2-METHOXY-NAPHT-1-YL)ETHYL]-CYCLOHEXYLCARBOXAMIDE

### EXEMPLE 13 : N-[2-(2,5,6-TRIMETHOXY-NAPHT-1-YL)ETHYL]-ACETAMIDE

En procédant comme dans l'exemple 1 mais en remplaçant la 2-(2-méthoxy-napht-1-yl)-éthylamine par la 2-(2,5,6-triméthoxy-napht-1-yl)-éthylamine (préparation 2), on obtient le composé du titre.

### EXEMPLES 14 A 18 :

En procédant comme dans l'exemple 1, mais en remplaçant successivement la 2-(2-méthoxy-napht-1-yl)-éthylamine par les composés des préparations 3 à 7, on obtient les composés des exemples suivants :

### EXEMPLE 14 : N-[2-(6-HYDROXY-2-METHOXY-NAPHT-1-YL)-ETHYL]-ACETAMIDE

### EXEMPLE 15 : N-[2-(2,6-DIPROPOXY-NAPHT-1-YL)-ETHYL]-ACETAMIDE

### EXEMPLE 16 : N-[2-(5,6,7,8-TETRAHYDRO-4-METHYL-2-METHOXY-NAPHT-1-YL)-ETHYL]-ACETAMIDE

### EXEMPLE 17 : N-[2-(6-METHOXY-1,4-BENZODIOXAN-5-YL)-ETHYL]-ACETAMIDE

### EXEMPLE 18 : N-[2-(6-METHOXY-3-METHYL-BENZOFURAN-7-YL)-ETHYL]-ACETAMIDE

### EXEMPLE 19 : N-[2-(5-METHOXY-INDOL-4-YL)-ETHYL]-ACETAMIDE

En procédant comme dans l'exemple 1, mais en remplaçant la 2-(2-méthoxy-napht-1-yl)-éthylamine par la 2-(5-méthoxy-indol-4-yl)-éthylamine (Bull. Soc. Chim. Fr. 1973, (6) (Pt. 2), pp 2046-2057), on obtient le composé du titre.

### EXEMPLES 20 A 22 :

En procédant comme dans les exemples 2 à 4, mais en remplaçant la 2-(2-méthoxy-napht-1-yl)-éthylamine par la 2-(5-méthoxy-indol-4-yl)-éthylamine et en utilisant les chlorures ou anhydrides appropriés, on obtient successivement les composés des exemples suivants :

### EXEMPLE 20 : N-[2-(5-METHOXY-INDOL-4-YL)-ETHYL]-PROPIONAMIDE

### EXEMPLE 21 : N-[2-(5-METHOXY-INDOL-4-YL)-ETHYL]-CYCLOPROPYLCARBOXAMIDE

### EXEMPLE 22 : N-[2-(5-METHOXY-INDOL-4-YL)-ETHYL]-TRIFLUOROACETAMIDE

### EXEMPLE 23 : N-[2-(2-HYDROXY-5-NITRO-PHENYL)-ETHYL]-CYCLOPROPYLCARBOXAMIDE

En procédant comme dans l'exemple 3, mais en remplaçant la 2-(2-méthoxy-napht-1-yl)-éthylamine par la 2-(2-hydroxy-5-nitro-phényl)-éthylamine (Tetrahedron Letters 1990, vol. 31, N° 9, pp 1275-1278), on obtient le composé du titre.

### EXEMPLE 24 : N-[2-(5-BROMO-2-METHOXY-PHENYL)-ETHYL]-CYCLOPROPYLCARBOXAMIDE

En procédant comme dans l'exemple 3, mais en remplaçant la 2-(2-méthoxy-napht-1-yl)-éthylamine par la 2-(5-bromo-2-méthoxy-phényl)-éthylamine (préparation 8), on obtient le composé du titre.

### EXEMPLES 25 A 27 :

En procédant comme dans l'exemple 24 mais en utilisant les chlorures d'acyle appropriés, on obtient les composés des exemples suivants :

### EXEMPLE 25 : N-[2-(5-BROMO-2-METHOXY-PHENYL)-ETHYL]-CYCLOBUTYLCARBOXAMIDE

### EXEMPLE 26 : N-[2-(5-BROMO-2-METHOXY-PHENYL)-ETHYL]-CYCLOHEXYLCARBOXAMIDE

### EXEMPLE 27 : N-[2-(5-BROMO-2-METHOXY-PHENYL)-ETHYL]-CYCLOPENTYLPROPIONAMIDE

### EXEMPLE 28 : N-[2-(2-METHOXY-NAPHT-1-YL)-1-METHYL-ETHYL]ACETAMIDE

Dans un bicol de 100 cm³, on introduit 0,37 g (1,72.10⁻³ mol) de l'amine obtenue à la préparation 9, et 20 cm³ de dichlorométhane anhydre, sous atmosphère inerte. On agite et refroidit le mélange, puis on introduit 0,32 cm³ (2,29.10⁻³ mol) de triéthylamine séchée sur potasse et enfin 0,13 cm³ (1,84.10⁻³ mol) de chlorure d'acétyle. On enlève ensuite la glace et on laisse évoluer à température ambiante pendant 1/2 heure. On hydrolyse en versant le mélange dans un bain d'eau agité. Après séparation des deux phases, on lave deux fois la phase aqueuse par CH₂Cl₂, et on sèche les phases organiques jointes sur MgSO₄. Après évaporation à sec de ces dernières, le résidu est purifié par chromatographie sur colonne de silice (éluant MeOH/CH₂Cl₂ :2/98) et on récupère 0,4 g (1,55.10⁻³ mol) de cristaux blancs de l'acétamide attendu, soit un rendement de 90 %.
Point de fusion : 151 ± 3°C

### EXEMPLE 29 A 34

En procédant comme dans l'exemple 28, mais en remplaçant le chlorure d'acétyle par le chlorure d'acyle ou l'anhydride approprié, on obtient les composés des exemples suivants :

### EXEMPLE 29 : N-[2-(2-METHOXY-NAPHT-1-YL)-1-METHYL-ETHYL]PROPIONAMIDE

Point de fusion : 133 ± 3°C

| Analyse élémentaire : | |
|---|---|
| C théo = 75,24 % | C exp = 74,98 % |
| H théo = 7,80 % | H exp = 7,91 % |
| N théo = 5,16 % | N exp = 5,10 % |

### EXEMPLE 30 : N-[2-(2-METHOXY-NAPHT-1-YL)-1-METHYL-ETHYL] TRIFLUOROACETAMIDE

Point de fusion : 123 ± 3°C

| Analyse élémentaire : | |
|---|---|
| C théo = 61,73 % | C exp = 61,54 % |
| H théo = 5,18 % | H exp = 5,29 % |
| N théo = 4,50 % | N exp = 4,40 % |

### EXEMPLE 31 : N-[2-(2-METHOXY-NAPHT-1-YL)-1-METHYL-ETHYL] CYCLOPROPYLCARBOXAMIDE

### EXEMPLE 32 : N-[2-(2-METHOXY-NAPHT-1-YL)-1-METHYL-ETHYL]BUTYRAMIDE

### EXEMPLE 33 : N-[2-(2-METHOXY-NAPHT-1-YL)-1-METHYL-ETHYL]PENTANAMIDE

### EXEMPLE 34 : N-[2-(2-METHOXY-NAPHT-1-YL)-1-METHYL-ETHYL] CYCLOBUTYLCARBOXAMIDE

### EXEMPLE 35 : N-[2-(2-METHOXY-5-METHYL-PHENYL)ETHYL]ACETAMIDE

Dans un bicol de 100 cm³, on introduit 0,31 g (1,54.10⁻³ mol) du chlorhydrate de l'amine obtenue lors de la préparation 10 que l'on solubilise dans environ 15 cm³ d'eau. Le mélange étant agité, on ajoute 1,6 g (19,51.10⁻³ mol) d'acétate de sodium puis 3,6 cm³ (38,15.10⁻³ mol) d'anhydride acétique à la seringue. On laisse évoluer à température ambiante durant 30 min. Le produit formé est extrait de la phase aqueuse trois fois par CH₂Cl₂. Cette phase organique est alors lavée avec une solution saturée de NaHCO₃ puis par de l'eau, et enfin séchée sur MgSO₄ et évaporée à sec. Le produit brut obtenu est purifié par chromatographie sur une colonne de silice (éluant : 5 % MeOH, 95 % CH₂Cl₂) et l'on obtient alors 0,25 g (1,206.10⁻³ mol) de l'acétamide attendu soit un rendement de 79 %.
Point de fusion : 76 ± 3°C

| Analyse élémentaire : | |
|---|---|
| C théo = 69,54 % | C exp = 69,36 % |
| N théo = 6,76 % | N exp = 6,72 % |
| H théo = 8,27 % | H exp = 8,40 % |

### EXEMPLE 36 : N-[2-(2-METHOXY-5-METHYL-PHENYL)ETHYL]PROPIONAMIDE

- Passage à l'amine libre :
   Dans un erlen, on solubilise 0,32 g (1.59.10⁻³ mol) de chlorhydrate de l'amine obtenue lors de la préparation 10 avec de l'eau et on ajoute 2 équivalents de soude 2N soit 1,59 cm³. L'amine libre est extraite trois fois par CH₂Cl₂ ; la phase organique est lavée par de l'eau puis séchée sur MgSO₄ et enfin évaporée à sec.
- Acylation de l'amine :
   L'amine obtenue est introduite dans un tricol de 100 cm³, solubilisée dans 20 cm³ de dichlorométhane anhydre. Le mélange sous argon est agité et refroidi par un bain de glace. On introduit alors 0,33 cm³ (2,36.10⁻³ mol) de triéthylamine puis lentement 0,17 cm³ (1,96.10⁻³ mol) de chlorure de propionyle. On laisse ensuite évoluer à température ambiante durant 30 min. L'hydrolyse est effectuée en versant le mélange réactionnel dans de l'eau. Le produit organique attendu est extrait trois fois par du dichlorométhane, les phases organiques jointes sont séchées sur MgSO₄ puis évaporées à sec. Le résidu solide obtenu est purifié par chromatographie sur une colonne de silice (éluant : MeOH/CH₂Cl₂ : 5/95). On obtient alors 0,30 g (1,36.10⁻³ mol) du propionamide attendu (solide blanc), soit un rendement de 85 %.

Point de fusion : 70 ± 3°C

| Analyse élémentaire : | |
|---|---|
| C théo = 70,56 % | C exp = 70,32 % |
| H théo = 8,65 % | H exp = 8,79 % |
| N théo = 6,33 % | N exp = 6,24 % |

### EXEMPLE 37 : N-[2-(2-METHOXY-5-METHYL-PHENYL)ETHYL]TRIFLUOROACETAMIDE

Le passage du chlorhydrate à l'amine libre est réalisé de la même manière que dans l'exemple 36 à partir de 0,35 g (1,74.10⁻³ mol) de chlorhydrate. L'amine obtenue est introduite dans un tricoi de 100 cm³, solubilisée par 20 cm³ de dichlorométhane anhydre. Le mélange étant agité, sous atmosphère inerte et refroidi par un bain de glace, on introduit 0,14 cm3 (1,73.10⁻³ mol) de pyridine séchée sur potasse puis 0,29 cm³ (2,05.10⁻³ mol) d'anhydride trifluoroacétique. On laisse ensuite évoluer à température ambiante durant 30 min. On hydrolyse l'anhydride excédentaire en versant le mélange réactionnel dans de l'eau agitée, puis après séparation des deux phases, le produit organique attendu est extrait trois fois par du CH₂Cl₂. La phase organique résultante est lavée par une solution saturée de NaHCO₃, puis par de l'eau, séchée sur MgSO₄ et évaporée à sec. Le résidu obtenu est purifié par chromatographie sur colonne de silice (éluant : MeOH/CH₂Cl₂ : 2/98). On obtient finalement 0,34 g (1,30.10⁻³ mol) de l'amide attendu, soit un rendement de 75 %. Point de fusion : 77 ± 3°C

| Analyse élémentaire : | |
|---|---|
| C théo = 55,17 % | C exp = 55,27 % |
| H théo = 5,40 % | H exp = 5,46 % |
| N théo = 5,36 % | N exp = 5,30 % |

### EXEMPLE 38 A 40 :

En procédant comme dans l'exemple 36 mais en remplaçant le chlorure de propionyle par le chlorure d'acyle approprié, on obtient les composés des exemples suivants :

### EXEMPLE 38 : N-[2-(2-METHOXY-5-METHYL-PHENYL)ETHYL]BUTYRAMIDE

### EXEMPLE 39 : N-[2-(2-METHOXY-5-METHYL-PHENYL)ETHYL] CYCLOPROPYLCARBOXAMIDE

### EXEMPLE 40 : N-[2-(2-METHOXY-5-METHYL-PHENYL)ETHYL]HEXANAMIDE

### EXEMPLE 41 : N-[2-(-5-ETHYL-2-METHOXY-PHENYL)ETHYL]ACETAMIDE

Dans un tricol de 50 cm³, on introduit 68,8 mg (3,19.10⁻⁴ mol) de chlorhydrate de l'amine obtenue lors de la préparation 11 que l'on solubilise avec 10 cm³ d'eau, puis 0,4 g (4,88.10⁻³ mol) d'acétate de sodium. Le milieu réactionnel étant agité, on ajoute ensuite 0,88 cm³ (9,33.10⁻³ mol) d'anhydride acétique et on laisse évoluer à température ambiante durant 40 min. L'amide formé est extrait du milieu réactionnel trois fois par CH₂Cl₂. Cette phase organique est alors lavée par une solution saturée de NaHCO₃, puis par de l'eau, et enfin séchée sur MgSO₄ et évaporée à sec. Le résidu solide obtenu est purifié par chromatographie sur colonne de silice (éluant : MeOH-CH₂Cl₂ : 2-98) et on récupère 61,4 mg (2,78.10⁻⁴ mol) de l'amide attendu, soit un rendement de 87 %.
Point de fusion : 82 ± 3°C

| Analyse élémentaire : | |
|---|---|
| C théo = 70,56 % | C exp = 70,36 % |
| H théo = 8,65 % | H exp = 8,72 % |
| N théo = 6,33 % | N exp = 6,23 % |

### EXEMPLE 42 A 44 :

En procédant comme dans l'exemple 41 mais en remplaçant l'anhydride acétique par le chlorure d'acyle ou l'anhydride approprié, on obtient les composés des exemples suivants :

### EXEMPLE 42 : N-[2-(5-ETHYL-2-METHOXY-PHENYL)ETHYL]PROPIONAMIDE

### EXEMPLE 43 : N-[2-(5-ETHYL-2-METHOXY-PHENYL)ETHYL]CYCLOPROPYLCARBOXAMIDE

### EXEMPLE 44 : N-[2-(5-ETHYL-2-METHOXY-PHENYL)EHTYL]CYCLOBUTILCARBOXAMIDE

### ETUDE PHARMACOLOGIQUE :

### EXEMPLE A : ETUDE DE LA TOXICITE AIGUE

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL₅₀, entraînant la mort de 50 % des animaux, a été évaluée.
La DL₅₀ des produits testés est supérieure à 1000 mg.kg⁻¹ pour les composés de l'invention étudiés ce qui indique l'absence de toxicité des composés de l'invention

### EXEMPLE B : ETUDE DE LIAISON AUX RECEPTEURS DE LA MELATONINE

L'étude de l'affinité des composés de l'invention pour les récepteurs de la mélatonine est réalisée sur des extraits membranaires de cerveaux de poulet selon la méthode décrite par Yuan H. and Pang S.F. (Journal of Endocrinology, (1991), 128, page 475-482).

### PROTOCOLE:

Dissection des cerveaux de poulet.

Les cerveaux sont disséqués rapidement à 4°C et les structures sont congelées à - 80°C. Les structures congelées sont ensuite homogénéisées au Polytron dans 10 volumes tampon (Tris : 50 mM, pH : 7,4, à 25°C).
Après 2 centrifugations à 44 000 g pendant 25 min et à 4°C, les culots sont resuspendus dans 10 volumes du même tampon. La quantité de protéines est déterminée par la méthode Folin-Lowry en présence de sodium dodécyl sulfate (SDS). Les protéines sont ensuite fractionnées et congelées à -80°C.

### MESURE DE LA LIAISON AUX RECEPTEURS DE LA MELATONINE:

Chaque essai est réalisé dans 0,25 cm³ de tampon (Tris 50 nM, pH 7,4) et contient 0,15 mg de protéines préparées ci-dessus, 0,05 nM de 2-[I¹²⁵]-iodomélatonine, et les composés à tester à des concentrations variables.
L'incubation dure 60 min à 25°C, puis on filtre le milieu d'incubation sur Brandel Cell Harvester® avec des filtres GF/B (Whatman).
Les filtres sont rincés 3 fois par 4 cm³ de tampon (Tris 50 mM, pH 7,4).
La fixation non spécifique est déterminée en présence de mélatonine à 10⁻⁵ M.
La radioactivité est compté avec un compteur β.
Les essais sont réalisés en triplicate.

### RESULTATS :

Les composés de l'invention possèdent une très haute affinité sélective pour les récepteurs à la mélatonine, pouvant être supérieure à celle de la mélatonine elle-même.
Cette affinité remarquable n'est aucunement retrouvée pour des composés présentant dans leur structure un radical hydroxyle ou alcoxy en position méta ou para de la chaîne alkylamidique du noyau benzène. au lieu de la position ortho telle que revendiquée pour les composés de l'invention.

### EXEMPLE C : COMPOSITION PHARMACEUTIQUE : COMPRIMES

Comprimés dosés à 5 mg de N-[2-(2-méthoxy-napht-1-yl)-éthyl]-trifluoroacétamide

| | |
|---|---|
| N-[2-(2-méthoxy-napht-1-yl)-éthyl]-trifluoracétamide | 5 g |
| Amidon de blé | 15 g |
| Amidon de maïs | 15 g |
| Lactose | 15 g |
| Stéarate de magnésium | 2 g |
| Silice | 1 g |
| Hydroxypropyl cellulose | 2 g |

## Revendications

1. Composés de formule (I) : 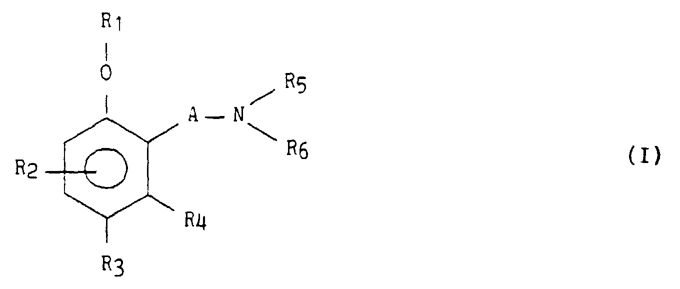 dans laquelle :
- A représente une chaîne -CH₂-CH₂- non substituée ou substituée par un alkyle inférieur,
- R₁ représente un atome d'hydrogène ou un alkyle inférieur,
- R₂ représente un atome d'hydrogène, un atome d'halogène, ou un alkyle inférieur,
- R₃ et R₄, identiques ou différents, représentent chacun indépendamment l'un de l'autre, un radical choisi parmi :
. hydrogène,
. halogène,
. alkyle inférieur,
. cyano,
. carboxyle,
. nitro,
. amino,
. alkylamino inférieur,
. dialkylamino inférieur,
. -(CH₂)ₙ-E₁ dans lequel n représente 0 ou un nombre entier de 1 à 4, et E₁ représente un cycloalkyle ou un cycloalcényle et contient de 3 à 8 atomes de carbone, E₁ étant non substitué ou substitué par un ou plusieurs radicaux choisis parmi halogène, oxo, et alkyle inférieur,
. et -(CH₂)_{n'}-E₂ dans lequel n' représente 0 ou un nombre entier de 1 à 4 et E₂ représente un radical choisi parmi : phényle, naphtyle, pyrrolyle, thiényle, furyle, pyridyle, pyrazinyle, pyrimidinyle, pyridazinyle, quinolyle, et isoquinolyle, E₂ étant non substitué ou substitué par un ou plusieurs radicaux choisis parmi : halogène, hydroxyle, alkyle inférieur, alcoxy inférieur, et trifluorométhyle,
ou R₃ et R₄ forment ensemble avec le noyau benzène qui les porte un système cyclique E₃ choisi parmi : indène, naphtalène, benzothiophène, benzofurane, indole, benzimidazole, benzopyrane, benzothiopyrane, quinoléine, isoquinoléine, indazole, quinoxaline, quinazoline, cinnoline, benzothiazole, benzisothiazole, benzoxazole, benzisoxazole, benzoxazine, benzothiazine, benzodioxole, et benzodioxane,
étant entendu que la partie du système cyclique E₃ formée par R₃ et R₄ et les 2 atomes de carbone du noyau benzène qui les portent est :
- non hydrogénée ou partiellement hydrogénée,
- et non substituée ou substituée par un ou plusieurs radicaux choisis parmi : halogène, hydroxyle, alkyle inférieur, alcoxy inférieur, alcoxycarbonyle inférieur, et trifluorométhyle,
- R₅ représente un atome d'hydrogène ou un alkyle inférieur,
- R₆ représente un groupement 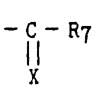 dans lequel X représente un atome de soufre ou d'oxygène et R₇ représente un radical choisi parmi :
. alkyle inférieur non substitué ou substitué par un ou plusieurs radicaux choisis parmi halogène, hydroxy, et alcoxy inférieur,
. alcènyle linéaire ou ramifié de 2 à 7 atomes de carbone non substitué ou substitué par un ou plusieurs radicaux choisis parmi halogène, hydroxy, et alcoxy inférieur,
. et -(CH₂)ₘ-E₄ dans lequel m représente 0 ou un nombre entier de 1 à 4 et E₄ représente un cycloalkyle, mono ou bicyclique, de 3 à 12 atomes de carbone non substitué ou substitué par un ou plusieurs radicaux choisis parmi halogène, oxo, et alkyle inférieur,
avec la réserve que R₇ peut représenter un alkyle inférieur non substitué ou substitué par un ou plusieurs radicaux choisis parmi halogène, hydroxy, et alcoxy inférieur, que si R₃ et R₄ forment avec le noyau benzène qui les porte un système cyclique E₃ tel que défini précédemment ou si un des substituants R₃ et R₄ représente un alkyle inférieur et que l'autre des substituants R₃ et R₄ représente un atome d'hydrogène,
leurs isomères optiques,
et leurs sels d'addition à un acide ou à une base pharmaceutiquement acceptable lorsque R₃ ou R₄ représente un groupement salifiable,
étant entendu que les termes "alkyle inférieur" et "alcoxy inférieur" désignent des groupements linéaires ou ramifiés de 1 à 6 atomes de carbone et que les termes "cycloalcènyle" et "alcènyle" désignent des groupements hydrocarbonés contenant une ou plusieurs double liaisons.

2. Composés selon la revendication 1 dans lesquels R₁ représente un radical méthyle.

3. Composés selon la revendication 1 dans lesquels R₃ et R₄ forment ensemble avec le noyau benzène qui les porte un système cyclique E₃ choisi parmi : indène, naphtalène, benzothiophène, benzofurane, indole, benzimidazole, benzopyrane, benzothiopyrane, quinoléine, isoquinoléine, indazole, quinoxaline, quinazoline, cinnoline, benzothiazole, benzisothiazole, benzoxazole, benzisoxazole, benzoxazine, benzothiazine, benzodioxole, et benzodioxane,
étant entendu que la partie du système cyclique E₃ formée par R₃ et R₄ et les 2 atomes de carbone du noyau benzène qui les portent est :
- non hydrogénée ou partiellement hydrogénée,
- et non substituée ou substituée par un ou plusieurs radicaux choisis parmi : halogène, hydroxyle, alkyle inférieur, alcoxy inférieur, alcoxycarbonyle inférieur, et trifluorométhyle.

4. Composés selon la revendication 1 dans lesquels R₃ et R₄ forment ensemble avec le noyau benzène qui les porte un naphtalène,
étant entendu que le noyau benzène formé par R₃, R₄, et les 2 atomes de carbone qui les portent est non substitué ou substitué par un ou plusieurs radicaux choisis parmi halogène, hydroxyle, alkyle inférieur, alcoxy inférieur, alcoxycarbonyle inférieur et trifluorométhyle.

5. Composés selon la revendication 1 dans lesquels R₁ représente un radical méthyle, et R₃ et R₄ forment ensemble avec le noyau benzène qui les porte un naphtalène étant entendu que le noyau benzène formé par R₃ et R₄ et les 2 atomes de carbone qui les portent est non substitué ou substitué par un ou plusieurs radicaux choisis parmi : halogène, hydroxyle, alkyle inférieur, alcoxy inférieur, alcoxycarbonyle inférieur, et trifluorométhyle.

6. Composé selon la revendication 1 qui est le N-[2-(2-méthoxy-napht-1-yl)-éthyl]-acétamide.

7. Composé selon la revendication 1 qui est le N-[2-(2-méthoxy-napht-1-yl)-éthyl]-propionamide.

8. Composé selon la revendication 1 qui est le N-[2-(2-méthoxy-napht-1-yl)-éthyl]-cyclopropylcarboxamide.

9. Composé selon la revendication 1 qui est le N-[2-(2-méthoxy-napht-1-yl)-éthyl]-trifluoroacétamide.

10. Composé selon la revendication 1 qui est le N-[2-(5-éthyl-2-méthoxyphényl)-éthyl]propionamide.

11. Procédé de préparation des composés de formule (I), selon la revendication 1, caractérisé en ce que :
on fait réagir un composé de formule (II) : 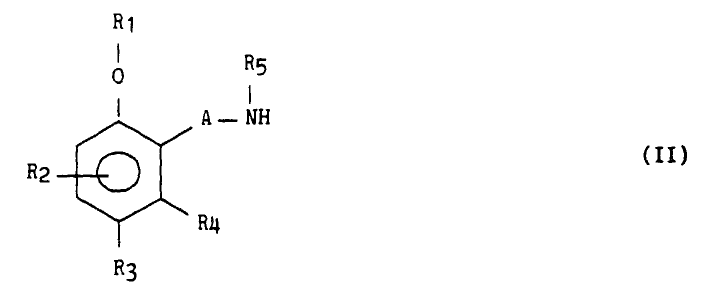 dans laquelle A, R₁, R₂, R₃, R₄ et R₅ sont tels que définis dans la revendication 1,
avec un composé de formule (III) : 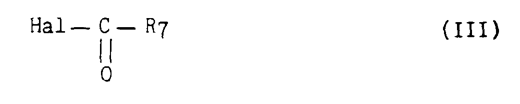 ou de formule (IV) : 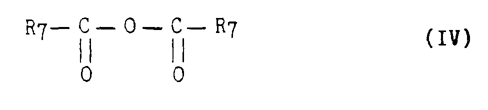 dans lesquelles R₇ est tel que défini dans la revendication 1 et Hal représente un atome d'halogène, pour obtenir un composé de formule (I/a) : 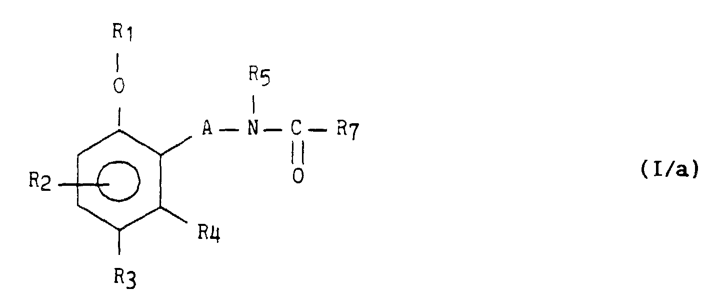 dans laquelle A, R₁, R₂, R₃, R₄, R₅, et R₇ sont tels que définis précédemment,
composé de formule (I/a) qui est soumis au réactif de Lawesson afin d'obtenir le composé de formule (I/b) : 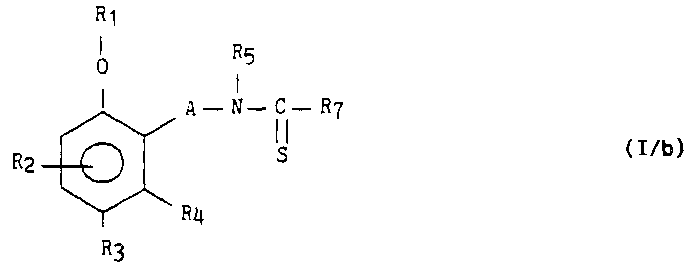 dans laquelle A, R₁, R₂, R₃, R₄, R₅, et R₇ sont tels que définis précédemment,
les composés de formule (I/a) et (I/b) formant l'ensemble des composés de formule (I) selon la revendication 1,
les composés de formule (I) pouvant être :
- purifiés suivant une ou plusieurs méthodes de purification choisies parmi la cristallisation, la chromatographie sur colonne de silice, la chromatographie en phase gazeuse, l'extraction, la filtration, et le passage sur charbon ou résine,
- séparés, le cas échéant, sous forme pure ou sous forme de mélange, en leurs éventuels isomères optiques,
- ou salifiés par un acide ou une base pharmaceutiquement acceptable.

12. Composition pharmaceutique contenant comme principe actif au moins un des composés selon la revendication 1, en combinaison avec un ou plusieurs excipients pharmaceutiquement acceptables.

13. Composition pharmaceutique selon la revendication 12 utile dans la prévention ou le traitement des troubles du système mélatoninergique.

## Claims

1. Compounds of formula (I): 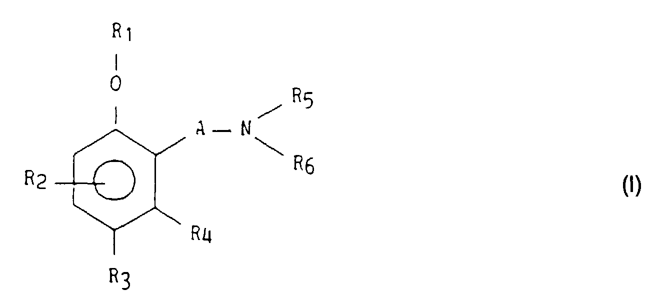 in which:
- A represents a chain -CH₂-CH₂- that is unsubstituted or substituted by a lower alkyl,
- R₁ represents a hydrogen atom or a lower alkyl,
- R₂ represents a hydrogen atom, a halogen atom or a lower alkyl,
- R₃ and R₄, which are identical or different, each represent, independently of the other, a radical selected from:
• hydrogen,
• halogen,
• lower alkyl,
• cyano,
• carboxy,
• nitro,
• amino,
• lower alkylamino,
• di-lower alkylamino,
• -(CH₂)ₙ-E₁ in which n represents 0 or an integer from 1 to 4 and E₁ represents a cycloalkyl or a cycloalkenyl and contains from 3 to 8 carbon atoms, E₁ being unsubstituted or substituted by one or more radicals selected from halogen, oxo and lower alkyl,
• and -(CH₂)_{n'}-E₂ in which n' represents 0 or an integer from 1 to 4 and E₂ represents a radical selected from: phenyl, naphthyl, pyrrolyl, thienyl, furyl, pyridyl, pyrazinyl, pyrimidinyl, pyridazinyl, quinolyl and isoquinolyl, E₂ being unsubstituted or substituted by one or more radicals selected from: halogen, hydroxy, lower alkyl, lower alkoxy and trifluoromethyl,
or R₃ and R₄ form together with the benzene ring carrying them a cyclic system E₃ selected from: indene, naphthalene, benzothiophene, benzofuran, indole, benzimidazole, benzopyran, benzothiopyran, quinoline, isoquinoline, indazole, quinoxaline, quinazoline, cinnoline, benzothiazole, benzisothiazole, benzoxazole, benzisoxazole, benzoxazine, benzothiazine, benzodioxole and benzodioxane, wherein the part of the cyclic system E₃ formed by R₃ and R₄ and the 2 carbon atoms of the benzene ring carrying them is:
- not hydrogenated or partially hydrogenated,
- and unsubstituted or substituted by one or more radicals selected from: halogen, hydroxy, lower alkyl, lower alkoxy, lower alkoxycarbonyl and trifluoromethyl,
- R₅ represents a hydrogen atom or a lower alkyl,
- R₆ represents a group 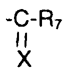 in which X represents a sulphur or oxygen atom and R₇ represents a radical selected from:
• lower alkyl that is unsubstituted or substituted by one or more radicals selected from halogen, hydroxy and lower alkoxy,
• linear or branched alkenyl having from 2 to 7 carbon atoms that is unsubstituted or substituted by one or more radicals selected from halogen, hydroxy and lower alkoxy,
• and -(CH₂)ₘ-E₄ in which m represents 0 or an integer from 1 to 4 and E₄ represents a mono- or bi-cyclic cycloalkyl having from 3 to 12 carbon atoms that is unsubstituted or substituted by one or more radicals selected from halogen, oxo and lower alkyl,
with the proviso that R₇ may represent a lower alkyl that is unsubstituted or substituted by one or more radicals selected from halogen, hydroxy and lower alkoxy, only when R₃ and R₄ form with the benzene ring carrying them a cyclic system E₃ as defined above or when one of the substituents R₃ and R₄ represents a lower alkyl and the other substituent R₃ or R₄ represents a hydrogen atom,
their optical isomers,
and their addition salts with a pharmaceutically acceptable acid or base when R₃ or R₄ represents a salt-forming group,
wherein the terms "lower alkyl" and "lower alkoxy" denote linear or branched groups having from 1 to 6 carbon atoms and the terms "cycloalkenyl" and "alkenyl" denote hydrocarbon groups containing one or more double bonds.

2. Compounds according to claim 1 in which R₁ represents a methyl radical.

3. Compounds according to claim 1 in which R₃ and R₄ form together with the benzene ring carrying them a cyclic system E₃ selected from: indene, naphthalene, benzothiophene, benzofuran, indole, benzimidazole, benzopyran, benzothiopyran, quinoline, isoquinoline, indazole, quinoxaline, quinazoline, cinnoline, benzothiazole, benzisothiazole, benzoxazole, benzisoxazole, benzoxazine, benzothiazine, benzodioxole and benzodioxane,
wherein the part of the cyclic system E₃ formed by R₃ and R₄ and the 2 carbon atoms of the benzene ring carrying them is:
- not hydrogenated or partially hydrogenated,
- and unsubstituted or substituted by one or more radicals selected from: halogen, hydroxy, lower alkyl, lower alkoxy, lower alkoxycarbonyl and trifluoromethyl.

4. Compounds according to claim 1 in which R₃ and R₄ form together with the benzene ring carrying them a naphthalene radical,
wherein the benzene ring formed by R₃, R₄ and the 2 carbon atoms carrying them is unsubstituted or substituted by one or more radicals selected from halogen, hydroxy, lower alkyl, lower alkoxy, lower alkoxycarbonyl and trifluoromethyl.

5. Compounds according to claim 1 in which R₁ represents a methyl radical and R₃ and R₄ form together with the benzene ring carrying them a naphthalene radical,
wherein the benzene ring formed by R₃ and R₄ and the 2 carbon atoms carrying them is unsubstituted or substituted by one or more radicals selected from: halogen, hydroxy, lower alkyl, lower alkoxy, lower alkoxycarbonyl and trifluoromethyl.

6. Compound according to claim 1 which is N-[2-(2-methoxynaphth-1-yl)ethyl]-acetamide.

7. Compound according to claim 1 which is N-[2-(2-methoxynaphth-1-yl)ethyl]-propionamide.

8. Compound according to claim 1 which is N-[2-(2-methoxynaphth-1-yl)ethyl]-cyclopropylcarboxamide.

9. Compound according to claim 1 which is N-[2-(2-methoxynaphth-1-yl)ethyl]-trifluoroacetamide.

10. Compound according to claim 1 which is N-[2-(5-ethyl-2-methoxyphenyl)-ethyl]propionamide.

11. Process for the preparation of compounds of formula (I) according to claim 1, characterised in that:
a compound of formula (II): 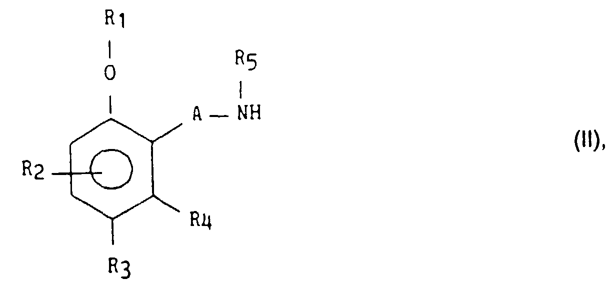 in which A, R₁, R₂, R₃, R₄ and R₅ are as defined in claim 1,
is reacted with a compound of formula (III): 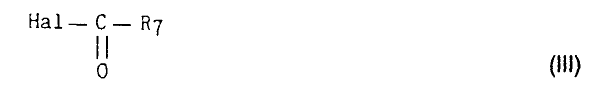 or of formula (IV): 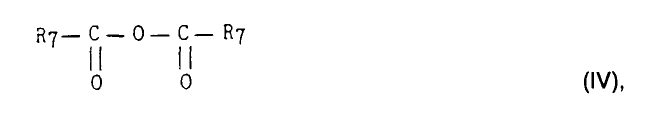 in which R₇ is as defined in claim 1 and Hal represents a halogen atom, to obtain a compound of formula (I/a): 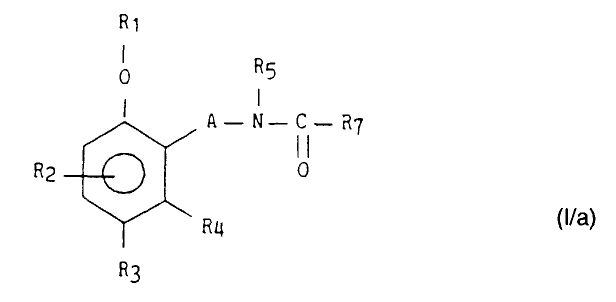 in which A, R₁, R₂, R₃, R₄, R₅ and R₇ are as defined above,
which compound of formula (I/a) is subjected to Lawesson's reagent in order to obtain the compound of formula (I/b): 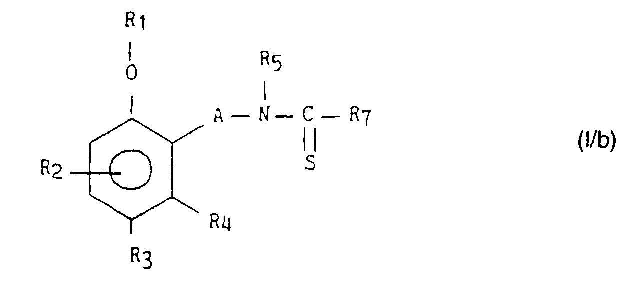 in which A, R₁, R₂, R₃, R₄ ,R₅ and R₇ are as defined above,
the compounds of formulae (I/a) and (I/b) forming the totality of the compounds of formula (I) according to claim 1,
which compounds of formula (I) may be:
- purified in accordance with one or more purification methods selected from crystallisation, chromatography on a silica column, gas chromatography, extraction, filtration, and passage over charcoal or resin,
- separated, where appropriate, in pure form or in the form of a mixture, into their possible optical isomers,
- or converted into a salt using a pharmaceutically acceptable acid or base.

12. Pharmaceutical composition comprising as active ingredient at least one of the compounds according to claim 1 in combination with one or more pharmaceutically acceptable excipients.

13. Pharmaceutical composition according to claim 12 for use in the prevention or treatment of disorders of the melatoninergic system.

## Patentansprüche

1. Verbindungen der Formel (I): 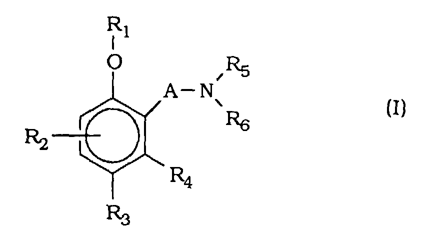 in der:
- A eine Kette -CH₂-CH₂-, die nichtsubstituiert oder durch eine Niedrigalkylgruppe substituiert ist,
- R₁ ein Wasserstoffatom oder eine Niedrigalkylgruppe,
- R₂ ein Wasserstoffatom, ein Halogenatom oder eine Niedrigalkylgruppe,
- R₃ und R₄, die gleichartig oder verschieden sein können, jeweils unabhängig voneinander eine Gruppe ausgewählt aus:
. Wasserstoff.
. Halogen,
. Niedrigalkyl,
. Cyano,
. Carboxyl,
. Nitro,
. Amino,
. Niedrigalkylamino,
. Niedrigdialkylamino,
. -(CH₂)ₙ-E₁, worin n 0 oder eine ganze Zahl mit einem Wert von 1 bis 4 und E₁ eine Cycloalkyl- oder Cycloalkenylgruppe, die 3 bis 8 Kohlenstoffatome enthält, darstellen, wobei E₁ nichtsubstituiert oder durch eine oder mehrere Gruppen ausgewählt aus Halogenatomen, Oxogruppen und Niedrigalkylgruppen substituiert ist,
. -(CH₂)_{n'}-E₂, worin n' 0 oder eine ganze Zahl mit einem Wert von 1 bis 4 und E₂ eine Gruppe ausgewählt aus: Phenyl, Naphthyl, Pyrrolyl, Thienyl, Furyl, Pyridyl, Pyrazinyl, Pyrimidinyl, Pyridazinyl, Chinolyl und Isochinolyl darstellen, wobei E₂ nichtsubstituiert oder durch eine oder mehrere Gruppen ausgewählt aus Halogen, Hydroxyl, Niedrigalkyl, Niedrigalkoxy und Trifluormethyl substituiert ist,
oder R₃ und R₄ gemeinsam mit dem sie tragenden Benzolkern ein cyclisches System E₃ bilden, ausgewählt aus: Inden, Naphthalin, Benzothiophen, Benzofuran, Indol, Benzimidazol, Benzopyran, Benzothiopyran, Chinolin, Isochinolin, Indazol, Chinoxalin, Chinazolin, Cinnolin, Benzothiazol, Benzisothiazol, Benzoxazol, Benzisoxazol, Benzoxazin, Benzothiazin, Benzodioxol und Benzodioxan, wobei es sich versteht, daß der Teil des cyclischen Systems E₃, der durch R₃ und R₄ und die sie tragenden beiden Kohlenstoffatome des Benzolkerns gebildet ist:
- nicht hydriert oder teilweise hydriert,
- und nichtsubstituiert oder durch eine oder mehrere Gruppen ausgewählt aus: Halogen, Hydroxyl, Niedrigalkyl, Niedrigalkoxy, Niedrigalkoxycarbonyl und Trifluormethyl substituiert ist,
- R₅ ein Wasserstoffatom oder eine Niedrigalkylgruppe,
- R₆ eine Gruppe 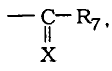 in der X ein Schwefelatom oder ein Sauerstoffatom und R₇ eine Gruppe ausgewählt aus:
. Niedrigalkyl, das nichtsubstituiert oder durch eine oder mehrere Gruppen ausgewählt aus Halogen, Hydroxyl und Niedrigalkoxy substituiert ist,
. geradkettige oder verzweigtes Alkenyl mit 2 bis 7 Kohlenstoffatomen, das nichtsubstituiert oder durch eine oder mehrere Gruppen ausgewählt aus Halogen, Hydroxyl und Niedrigalkoxy substituiert ist,
. und -(CH₂)ₘ-E₄, in der m 0 oder eine ganze Zahl mit einem Wert von 1 bis 4 und E₄ eine mono- oder bicyclische Cycloalkylgruppe mit 3 bis 12 Kohlenstoffatomen, die nichtsubstituiert oder durch eine oder mehrere Gruppen ausgewählt aus Halogen, Oxo und Niedrigalkyl substituiert ist, darstellen,
mit der Maßgabe bedeuten, daß R₇ nur dann eine Niedrigalkylgruppe, die nichtsubstituiert oder durch eine oder mehrere Gruppen ausgewählt aus Halogen, Hydroxyl und Niedrigalkoxy substituiert sein kann, darstellt, wenn R₃ und R₄ zusammen mit dem sie tragenden Benzolkern ein cyclisches System E₃ der oben definierten Art bilden, oder wenn einer Substituenten R₃ und R₄ eine Niedrigalkylgruppe und der andere der Substituenten R₃ und R₄ ein Wasserstoffatom bedeuten,
deren optische Isomeren und
deren Additionssalze mit einer pharmazeutisch annehmbaren Säure oder Base, wenn R₃ oder R₄ eine in die Salzform überführbare Gruppe darstellt,
wobei es sich versteht, daß die Begriffe "Niedrigalkyl" und "Niedrigalkoxy" geradkettige oder verzweigte Gruppen mit 1 bis 6 Kohlenstoffatomen und die Begriffe "Cycloalkenyl" und "Alkenyl" eine oder mehrere Doppelbindungen aufweisende Kohlenwasserstoffgruppen bedeuten.

2. Verbindungen nach Anspruch 1, worin R₁ eine Methylgruppe bedeutet.

3. Verbindungen nach Anspruch 1, worin R₃ und R₄ zusammen mit dem sie tragenden Benzolkern ein cyclisches System E₃ bilden ausgewählt aus: Inden, Naphthalin, Benzothiophen, Benzofuran, Indol, Benzimidazol, Benzopyran, Benzothiopyran, Chinolin, Isochinolin, Indazol, Chinoxalin, Chinazolin, Cinnolin, Benzothiazol, Benzisothiazol, Benzoxazol, Benzisoxazol, Benzoxazin, Benzothiazin, Benzodioxol und Benzodioxan,
wobei es sich versteht, daß der Teil des cyclischen Systems E₃, der durch R₃ und R₄ und die beiden Kohlenstoffatome des sie tragenden Benzolkerns gebildet wird:
- nicht hydriert oder teilweise hydriert,
- und nicht substituiert oder durch eine oder mehrere Gruppen ausgewählt aus: Halogen, Hydroxyl, Niedrigalkyl, Niedrigalkoxy, Niedrigalkoxycarbonyl und Trifluormethyl substituiert ist.

4. Verbindungen nach Anspruch 1, worin R₃ und R₄ gemeinsam mit dem sie tragenden Benzolkern einen Naphthalinrest bilden,
wobei es sich versteht, daß der durch R₃, R₄ und die beiden sie tragenden Kohlenstoffatome gebildete Benzolkern nicht substituiert oder durch eine oder mehrere Gruppen ausgewählt aus Halogen, Hydroxyl, Niedrigalkyl, Niedrigalkoxy, Niedrigalkoxycarbonyl und Trifluormethyl substituiert ist.

5. Verbindungen nach Anspruch 1, worin R₁ eine Methylgruppe darstellt und R₃ und R₄ gemeinsam mit dem sie tragenden Benzolkern einen Naphthalinrest bilden, wobei es sich versteht, daß der durch R₃ und R₄ und die beiden sie tragenden Kohlenstoffatome gebildete Benzolkern nicht substituiert oder durch eine oder mehrere Gruppen ausgewählt aus: Halogen, Hydroxyl, Niedrigalkyl, Niedrigalkoxy, Niedrigalkoxycarbonyl und Trifluormethyl substituiert ist.

6. Verbindung nach Anspruch 1, nämlich N-[2-(2-Methoxy-naphth-1-yl)-ethyl]-acetamid.

7. Verbindung nach Anspruch 1, nämlich N-[2-(2-Methoxy-naphth-1-yl)-ethyl]-propionamld.

8. Verbindung nach Anspruch 1, nämlich N-[2-(2-Methoxy-naphth-1-yl)-ethyl]-cyclopropylcarboxamid.

9. Verbindung nach Anspruch 1, nämlich N-[2-(2-Methoxy-naphth-1-yl)-ethyl]-trifluoracetamid.

10. Verbindung nach Anspruch 1, nämlich N-[2-(5-Ethyl-2-methoxy-phenyl)-ethyl]-propionamid.

11. Verfahren zur Herstellung der Verbindungen der Formel (I) nach Anspruch 1, **dadurch gekennzeichnet**, daß man
eine Verbindung derFormel (II): 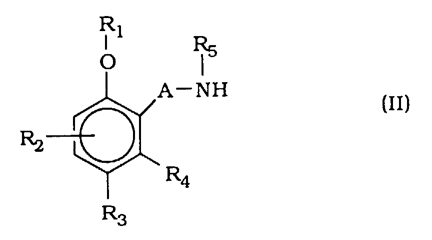 in der A, R₁, R₂, R₃, R₄ und R₅ die in Anspruch 1 angegebenen Bedeutungen besitzen,
mit einer Verbindung der Formel (III): 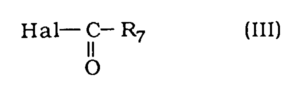 oder der Formel (IV): 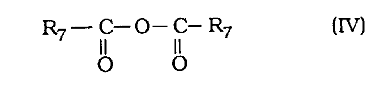 in denen R₇ die in Anspruch 1 angegebenen Bedeutungen besitzt und Hal ein Halogenatom darstellt, umsetzt zur Bildung einer Verbindung der Formel (I/a): 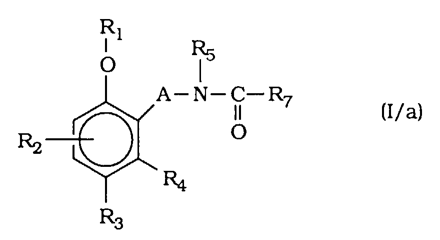 in der A, R₁, R₂, R₃, R₄, R₅ und R₇ die oben angegebenen Bedeutungen besitzen, welche Verbindung der Formel (I/a) der Einwirkung des Lawesson-Reagens unterworfen wird zur Bildung der Verbindung der Formel (I/b): 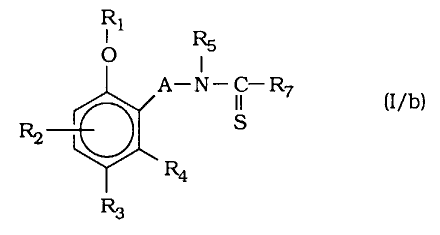 in der A, R₁, R₂, R₃, R₄, R₅ und R₇ die oben angegebenen Bedeutungen besitzen, welche Verbindungen der Formeln (I/a) und (I/b) gemeinsam die Verbindungen der Formel (I) von Anspruch 1 bilden,
welche Verbindungen der Formel (I):
- mit Hilfe einer oder mehrerer Reinigungsmethoden ausgewählt aus Kristallisation, Säulenchromatographie über Siliciumdioxid, Gasphasenchromatographie, Extraktion, Filtration und Überführung über Kohlenstoff oder ein Harz gereinigt,
- gegebenenfalls in reiner Form oder in Form einer Mischung in ihre etwaigen optischen Isomeren getrennt,
- oder mit einer pharmazeutisch annehmbaren Säure oder Base in ein Salz überführt werden können.

12. Pharmazeutische Zubereitung enthaltend als Wirkstoff mindestens eine der Verbindungen nach Anspruch 1 in Kombination mit einem oder mehreren pharmazeutisch annehmbaren Trägermaterialien.

13. Pharmazeutische Zubereitung nach Anspruch 12 zur Vorbeugung oder Behandlung von Störungen des melatoninergischen Systems.
